# EUROPEAN PATENT SPECIFICATION

(11) **EP 2 699 636 B1**
(45) Date of publication and mention of the grant of the patent: **30.09.2015**
(21) Application number: 12715097.7
(22) Date of filing: 17.04.2012
(51) Int. Cl.: C08L 43/04, C08F 290/06, C08K 5/05, C08L 91/00, A61K 8/91, A61Q 1/00, A61Q 19/00

(54) **COMPOSITIONS CONTAINING A POLYMER WITH A CARBOSILOXANE DENDRIMER UNIT AND A LARGE AMOUNT OF MONOALCOHOL**
ZUSAMMENSETZUNGEN MIT EINEM POLYMER MIT EINER CARBOSILOXANDENDRIMEREINHEIT UND HOHEM MONOALKOHOLGEHALT
COMPOSITIONS CONTENANT UN POLYMÈRE PRÉSENTANT UN MOTIF DENDRIMÈRE DE CARBOSILOXANE ET UNE GRANDE QUANTITÉ DE MONOALCOOL

(30) Priority: 22.04.2011 FR 1153497; 06.05.2011 US 201161483080 P
(43) Date of publication of application: 26.02.2014
(73) Proprietor: L'OREAL, 75008 Paris (FR)
(72) Inventor: ARNAUD, Pascal, F-94240 L'Hay Les Roses (FR)
(74) Representative: Domenego, Bertrand
(86) International application number: PCT/EP2012/056983
(87) International publication number: WO 2012/143344

(56) References cited:
- FR-A1- 2 935 269
- US-A1- 2005 008 597

## Description

The purpose of the invention is skincare and/or makeup compositions. More specifically, the invention relates to compositions that can be spread on the skin easily and quickly, and that are intended to confer an effect of mattness and that have an improved stability over time of the mattness.

Cosmetic compositions, such as, for example, foundations, are commonly used to give the skin, in particular the face, color and an esthetic effect. These makeup products generally contain oils, pigments, fillers and, optionally, additives such as cosmetic or dermatological active agents.

It is known to a person skilled in the art to use fillers to obtain a mattness effect. These fillers are most commonly selected according to their good properties of sebum absorption and/or to their ability to scatter light. However, their adhesion to the skin is generally weak, especially in the presence of sebum.

Film-forming polymers can then be used to improve the adhesion of these fillers, and to increase the stability of the mattness effect over the course of the day.

These polymers are of very different chemical natures and can be carried in the fatty phase or in the aqueous phase. By way of examples of these polymers, mention may be made of silicone resins, polyacrylates, latexes, etc.

Thus, US 6 887 859 describes skincare and makeup compositions containing a combination of film-forming polymers and fillers.

While these formulations actually make it possible to confer certain mattness stability properties on the cosmetic compositions, said properties may however be accompanied by unpleasant sensations and discomfort either during application of the product (difficult to spread, tacky effect, greasy feeling, etc.) or over the course of the day (tautness, mask effect, etc.).

FR 2 878 738 and EP 1 862 162 also describe cosmetic compositions containing a vinyl polymer comprising carbosiloxane dendrimer-derived units and fillers.

However, there remains the need for cosmetic compositions exhibiting a mattness and improved mattness stability, which are pleasant and easy to apply, while at the same time maintaining satisfactory application comfort, i.e. not causing a feeling of tautness or a mask effect throughout the day and/or not inducing a greasy or tacky feeling when applied.

Moreover, consumers are increasingly looking for cosmetic makeup or skincare products that can be spread easily and quickly on the skin in the form of a deposit that does not have to be thick, but that instead merges as much as possible with the support.

It is known to a person skilled in the art that liquid products with a high oil content enable good spreading and skin penetration properties to be obtained.

It is possible to cite, as examples of makeup products, liquid foundations, or in the field of skincare products, hydrating oily products and emollients or suntan oils.

This high oil content can, however, cause an oily and greasy sensation when applied, and lead to poor stability of the deposit on the skin over time.

It is therefore necessary to find a technical solution making it possible to obtain easy and agreeable spreading properties as well as properties of stability over time.

The purpose of the invention is to satisfy these needs.

This invention is therefore intended to provide a cosmetic composition with a good compromise between mattness and stability over time of said mattness, while maintaining satisfactory application comfort.

This invention is therefore intended to provide a cosmetic composition capable of being spread quickly and easily.

Thus, the invention relates to a composition including a physiologically acceptable medium containing at least one vinyl polymer having at least one carbosiloxane dendrimer-derived unit and one or more monoalcohol(s) containing 2 to 8 carbon atoms, in which the amount of monoalcohols ranges from 10% to 40% by weight with respect to the total weight of said composition.

The compositions according to the invention are cosmetic compositions intended for makeup and/or skincare.

In an unexpected manner, the inventors noted that the introduction, in a makeup and/or skincare composition, of a vinyl polymer having at least one carbosiloxane dendrimer-derived unit in combination with a high amount of monoalcohol(s) made it possible to confer very good mattness stability properties on these compositions while at the same time maintaining satisfactory application comfort. What is more, these compositions remain pleasant to wear throughout the day.

Until now, it had never been demonstrated that a combination of a polymer and monoalcohol(s) in a large amount according to the invention would make it possible to confer, on the cosmetic composition, mattness and improved mattness stability while conferring, in a very satisfactory manner, comfortable sensations during application of the composition and during its use throughout the day.

In an unexpected manner, it was noted by the inventors that the use of a high monoalcohol content made it possible to obtain products that can be spread easily and quickly, of which the application properties do not have a greasy sensation and of which the deposit has very good stability. Indeed, until now, monoalcohols were used in cosmetic compositions for skincare and/or makeup in small amounts, namely in amounts of less than 10% by weight.

### Vinyl polymer grafted with a carbosiloxane dendrimer

A vinyl polymer suitable for the preparation of a composition according to the invention comprises at least one carbosiloxane dendrimer-derived unit.

The vinyl polymer has a backbone and at least one side chain, which comprises a carbosiloxane dendrimer-derived unit having a carbosiloxane dendrimer structure.

The term "carbosiloxane dendrimer structure" in the context of the present invention represents a molecular structure possessing branched groups having high molecular masses, with said structure having a high regularity in the radial direction starting from the linkage to the backbone. Such carbosiloxane dendrimer structures are described in the form of a highly branched siloxane-silylalkylene copolymer in the Japanese patent application made available to public inspection Kokai 9-171 154.

A vinyl polymer according to the invention may contain carbosiloxane dendrimer-derived units, which can be represented by the following general formula (I): 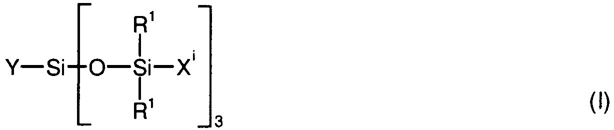 in which:
- R¹ represents an aryl group having from 5 to 10 carbon atoms or an alkyl group having from 1 to 10 carbon atoms;
- X¹ represents a silylalkyl group which, when i = 1, is represented by the formula (II):
   in which: 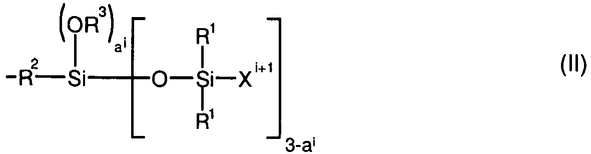
   . R¹ is as defined above in formula (I),
   . R² represents an alkylene group having from 2 to 10 carbon atoms,
   . R³ represents an alkyl group having from 1 to 10 carbon atoms,
   . Xⁱ⁺ⁱ is chosen from: a hydrogen atom, an alkyl group having from 1 to 10 carbon atoms, an aryl group having from 5 to 10 carbon atoms and a silylalkyl group as defined above of formula (II) with i = i + 1,
   . i is an integer from 1 to 10 that represents the generation of said silylalkyl group, and
   . aⁱ is an integer from 0 to 3;
- Y represents a radical-polymerizable organic group selected from:
   . organic groups containing a methacrylic group or an acrylic group and which are represented by the formulas: 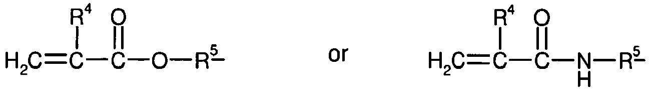 in which:
      * R⁴ represents a hydrogen atom or an alkyl group having from 1 to 10 carbon atoms; and
      * R⁵ represents an alkylene group having from 1 to 10 carbon atoms, such as a methylene group, an ethylene group, a propylene group or a butylene group, with the methylene group and the propylene group being preferred; and
   . organic groups containing a styryl group and which are represented by the formula: 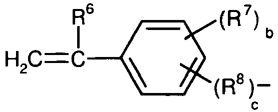 in which:
      * R⁶ represents a hydrogen atom or an alkyl group having from 1 to 10 carbon atoms, such as a methyl group, an ethyl group, a propyl group or a butyl group, with the methyl group being preferred;
      * R⁷ represents an alkyl group having from 1 to 10 carbon atoms;
      * R⁸ represents an alkylene group having from 1 to 10 carbon atoms, such as a methylene group, an ethylene group, a propylene group or a butylene group, with the ethylene group being preferred;
      * b is an integer from 0 to 4; and
      * c is 0 or 1, so that if c is 0, -(R⁸)_{c}- represents a bond.

According to an embodiment, R¹ may represent an aryl group having from 5 to 10 carbon atoms or an alky group having from 1 to 10 carbon atoms. The alkyl group may preferably be represented by a methyl group, an ethyl group, a propyl group, a butyl group, a pentyl group, an isopropyl group, an isobutyl group, a cyclopentyl group or a cyclohexyl group. The aryl group may preferably be represented by a phenyl group and a naphthyl group. The methyl and phenyl groups are more specifically preferred, and the methyl group is preferred above all.

According to an embodiment, R² represents an alkylene group having from 2 to 10 carbon atoms, in particular a linear alkylene group, such as an ethylene, propylene, butylene or hexylene group; or a branched alkylene group, such as a methylmethylene, methylethylene, 1-methylpentylene or 1,4-dimethylbutylene group.

The ethylene, methylethylene, hexylene, 1-methylpentylene and 1,4-dimethylbutylene groups are preferred above all.

According to an embodiment, R³ is chosen from the methyl, ethyl, propyl, butyl and isopropyl groups.

In formula (II), i indicates the number of generations and thus correspo9nds to the number of repetitions of the silylalkyl group.

For example, when the generation number is equal to one, the carbosiloxane dendrimer may be represented by the general formula shown below, in which Y, R¹, R² and R³ are as defined above, R¹² represents a hydrogen atom or is identical to R¹; a¹ is identical to aⁱ. Preferably, the mean total number of OR³ groups in a molecule is within the range of 0 to 7.

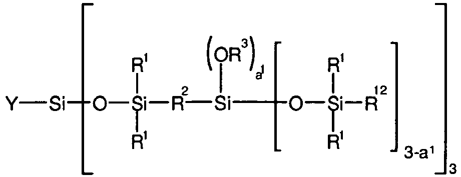

When the generation number is equal to 2, the carbosiloxane dendrimer may be represented by the second general formula shown below, in which Y, R¹, R², R³ and R¹² are the same as defined above; a¹ and a² represent the aⁱ of the indicated generation. Preferably, the mean total number of OR³ groups in a molecule is within the range of 0 to 25.

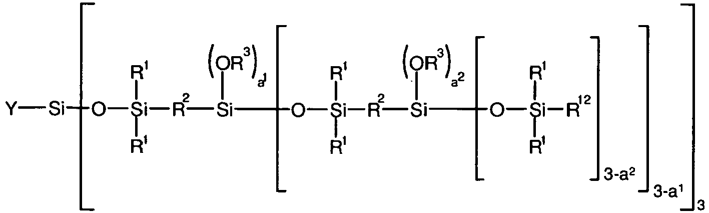

When the generation number is equal to 3, the carbosiloxane dendrimer is represented by the general formula shown below, in which Y, R¹, R², R³ and R¹² are the same as defined above; a¹, a² and a³ represent the aⁱ of the indicated generation. Preferably, the mean total number of OR³ groups in a molecule is within the range of 0 to 79.

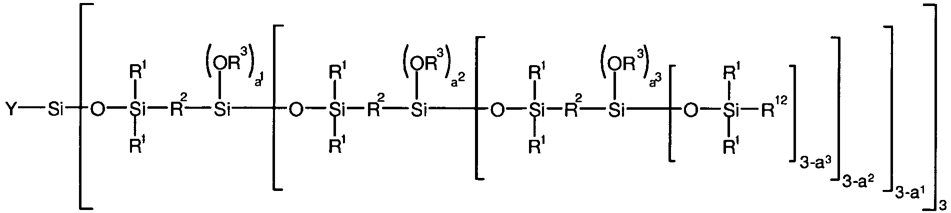

A vinyl polymer having at least one carbosiloxane dendrimer-derived unit has a side molecular chain containing a carbosiloxane dendrimer structure, and can result from the polymerization of:
(A) 0 to 99.9 parts by weight of a vinyl monomer; and
(B) 100 to 0.1 parts by weight of a carbosiloxane dendrimer containing a radical-polymerizable organic group, represented by the general formula (I) as defined above.

The vinyl monomer that is component (A) in the vinyl polymer having at least one carbosiloxane dendrimer-derived unit is a vinyl monomer that contains a radical-polymerizable vinyl group.

There is no particular limitation with regard to such a monomer.

The following are examples of this vinyl monomer: methyl methacrylate, ethyl methacrylate, n-propyl methacrylate, isopropyl methacrylate, or a similar lower alkyl methacrylate; glycidyl methacrylate; butyl methacrylate, butyl acrylate, n-butyl methacrylate, isobutyl methacrylate, tert-butyl acrylate, tert-butyl methacrylate, n-hexyl methacrylate, cyclohexyl methacrylate, 2-ethylhexyl acrylate, 2-ethylhexyl methacrylate, octyl methacrylate, lauryl methacrylate, stearyl acrylate, stearyl methacrylate, or a similar higher methacrylate; vinyl acetate, vinyl propionate, or a similar lower fatty acid vinyl ester; vinyl caproate, vinyl 2-ethylhexanoate, vinyl laurate, vinyl stearate, or a similar higher fatty acid ester; styrene, vinyltoluene, benzyl methacrylate, phenoxyethyl methacrylate, vinylpyrrolidone, and other similar vinylaromatic monomers; methacrylamide, N-methylolmethacrylamide, N-methoxymethylmethacrylamide, isobutoxymethoxyacrylamide, N,N-dimethylmethacrylamide, or similar vinyl monomers that contain amide groups; hydroxyethyl methacrylate, hydroxypropyl methacrylate, or similar vinyl monomers that contain hydroxyl groups; acrylic acid, methacrylic acid, itaconic acid, crotonic acid, fumaric acid, maleic acid, and similar vinyl monomers that contain a carboxylic acid group; tetrahydrofurfuryl methacrylate, butoxyethyl methacrylate, ethoxydiethylene glycol methacrylate, polyethylene glycol methacrylate, polypropylene glycol monomethacrylate, hydroxybutyl vinyl ether, cetyl vinyl ether, 2-ethylhexyl vinyl ether, or a similar vinyl monomer with ether bonds; methacryloxypropyltrimethoxysilane, polydimethyl-siloxanes containing a methacrylic group at one of its molecular ends, polydimethylsiloxane containing a styryl group at one of its molecular ends or a similar silicone compound containing unsaturated groups; butadiene; vinyl chloride; vinylidene chloride, methacrylonitrile; dibutyl fumarate; maleic anhydride; succinic acid anhydride; methacrylic glycidyl ether, an organic amine salt, an ammonium salt, and an alkaline metal salt of methacrylic acid, of itaconic acid, of crotonic acid, of maleic acid, or of fumaric acid; a radical-polymerizable unsaturated monomer having a sulfonic acid group such as a sulfonic acid styrene group; a quaternary ammonium salts derived from methacrylic acid, such as 2-hydroxy-3-methacryloxypropyltrimethylammonium chloride; and a methacrylic acid ester of an alcohol having a tertiary amine group, such as an ester of methacrylic acid and of diethylamine.

Multifunctional vinyl monomers can also be used.

The following are examples of such compounds: trimethylolpropane triacrylate, pentaerythritol trimethacrylate, ethylene glycol dimethacrylate, tetraethylene glycol dimethacrylate, polyethylene glycol dimethacrylate, 1,4-butanediol dimethacrylate, 1,6-hexanediol dimethacrylate, neopentyl glycol dimethacrylate, trimethylolpropanetrioxyethyl methacrylate, tris-(2-hydroxyethyl)isocyanurate dimethacrylate, tris-(2-hydroxyethyl)isocyanurate trimethacrylate, polydimethylsiloxane capped with styryl groups containing divinylbenzene groups at the two ends, or similar silicone compounds containing unsaturated groups.

A carbosiloxane dendrimer, which is component (B), may be represented by formula (I) as defined above.

The following represents preferred examples of group Y of formula (I): an methacryloxy group, a 3-acryloxypropyl group, a methacryloxymethyl group, a 3-methacryloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group, and a 5-hexenyl group.

A carbosiloxane dendrimer according to the present invention may be represented by the formulas with the following mean structures:

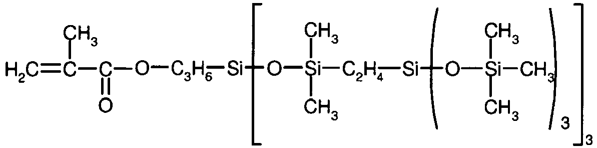

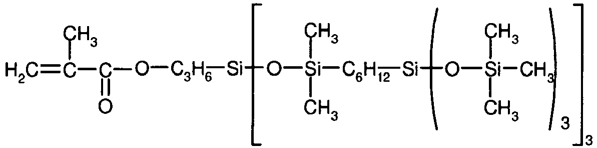

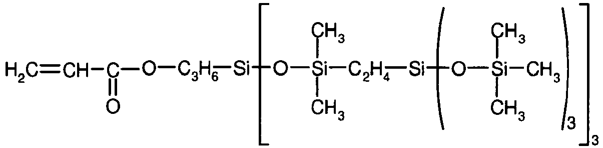

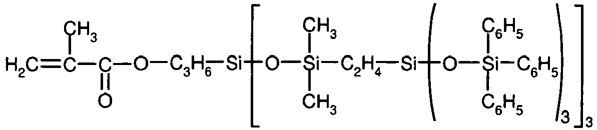

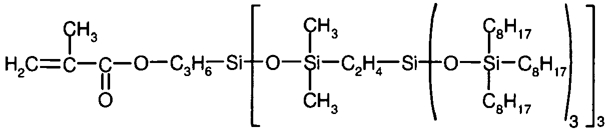

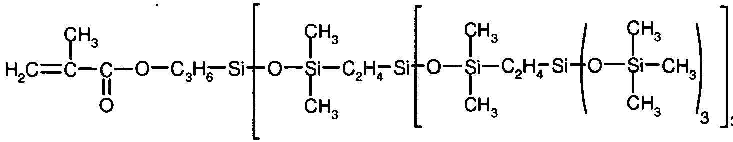

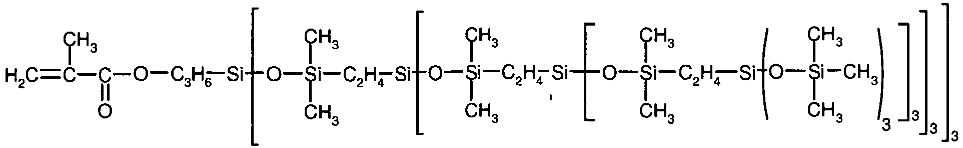

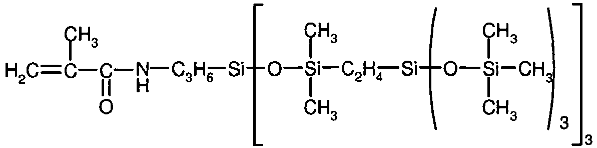

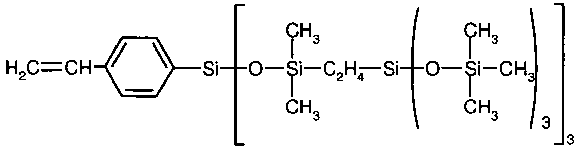

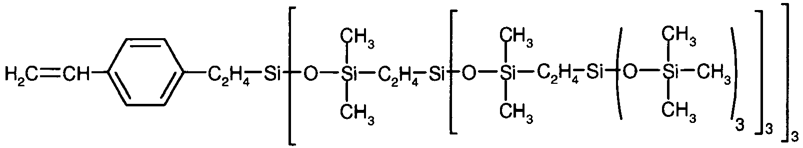

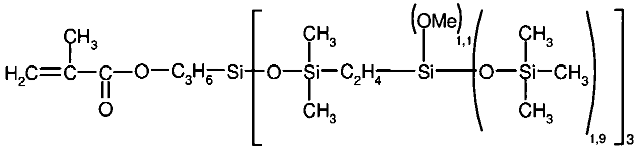

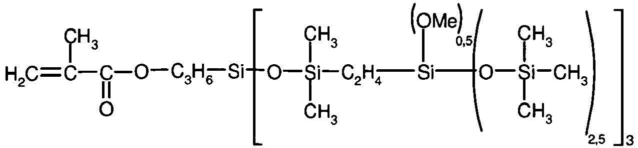

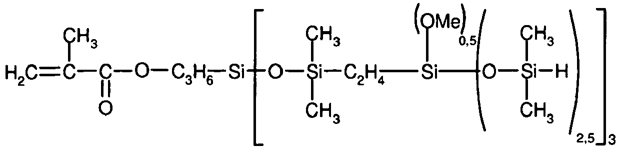

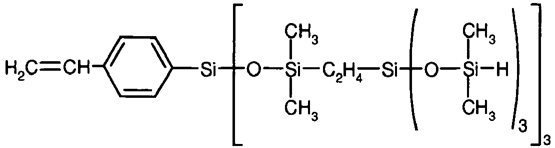

Thus, according to an embodiment, the carbosiloxane dendrimer of the composition of the present invention is represented by the following formula:

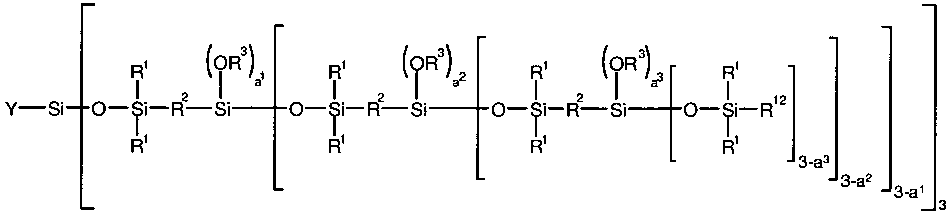

In which:
- Y, R¹, R² and R³ are as defined in formulas (I) and (II) above;
- a¹, a² and a³ satisfy the definition of aⁱ according to formula (II); and
- R¹² is H, an aryl group having from 5 to 10 carbon atoms or an alkyl group having from 1 to 10 carbon atoms.

According to an embodiment, the carbosiloxane dendrimer of the composition of the invention is represented by one of the following formulas:

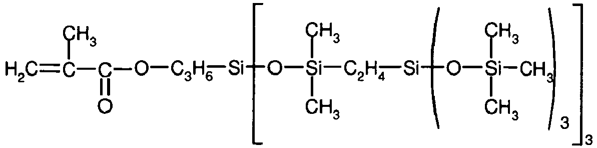

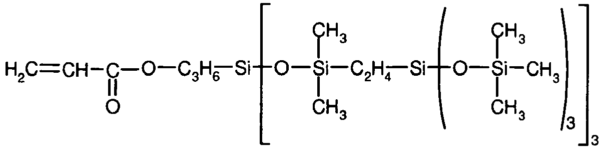

The vinyl polymer containing the carbosiloxane dendrimer according to the invention can be produced according to the method for producing a branched silalkylene siloxane described in the Japanese patent application Hei 9-171 154.

For example, it may be produced by subjecting an organosilicon compound containing a hydrogen atom bonded to a silicon atom to a hydrosilylation reaction, represented by the following general formula (IV): 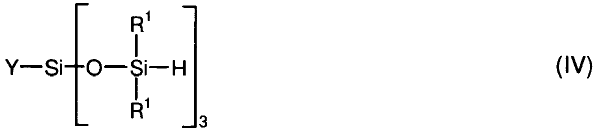 R¹ is as defined above in formula (I),
and an organosilicon compound containing an alkenyl group.

In the formula above, the organosilicon compound may be represented by 3-methacryloxypropyltris-(dimethylsiloxy) silane, 3-acryloxypropyltris-(dimethylsiloxy) silane, and 4-vinylphenyltris-(dimethylsiloxy)silane. The organosilicon compound that contains an alkenyl group may be represented by vinyltris-(trimethylsiloxy) silane, vinyltris-(dimethylphenylsiloxy) silane, and 5-hexenyltris-(trimethylsiloxy) silane.

The hydrosilylation reaction is produced in the presence of chloroplatinic acid, a vinylsiloxane and platinum complex, or a similar transition metal catalyst.

A vinyl polymer having at least one carbosiloxane dendrimer-derived unit may be chosen from among the polymers so that the carbosiloxane dendrimer-derived unit is a carbosiloxane dendritic structure represented by the formula (III): 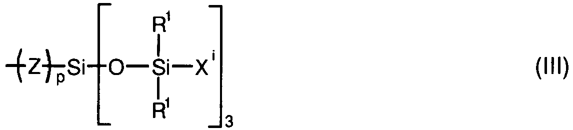 in which Z is a divalent organic group, "p" is 0 or 1, R¹ is as defined above in formula (IV) and Xⁱ is a silylalkyl group represented by formula (II) as defined above.

In a vinyl polymer having at least one carbosiloxane dendrimer-derived unit, the polymerization ratio between the components (A) and (B), in terms of the weight ratio between (A) and (B), is within a range from 0/100 to 99.9/0.1, or even 0.1/99.9 to 99.9/0.1, and preferably within a range from 1/99 to 99/1. A ratio between the components (A) and (B) of 0/100 means that the compound becomes a homopolymer of component (B).

A vinyl polymer having at least one carbosiloxane dendrimer-derived unit may be obtained by copolymerization of the components (A) and (B), or by polymerization of the component (B) alone.

The polymerization may be a radical polymerization or an ionic polymerization, however the radical polymerization is preferred.

The polymerization may be carried out by bringing about a reaction between the components (A) and (B) in a solution for a period of from 3 to 20 hours in the presence of a radical initiator at a temperature of from 50°C to 150°C.

A suitable solvent for this purpose is hexane, octane, decane, cyclohexane or a similar aliphatic hydrocarbon; benzene, toluene, xylene or a similar aromatic hydrocarbon; diethyl ether, dibutyl ether, tetrahydrofuran, dioxane, or similar ethers; acetone, methyl ethyl ketone, methyl isobutyl ketone, diisobutyl ketone, or similar ketones; methyl acetate, ethyl acetate, butyl acetate, isobutyl acetate or similar esters; methanol, ethanol, isopropanol, butanol or similar alcohols; octamethylcyclotetrasiloxane, decamethylcyclopentasiloxane, hexamethyldisiloxane, octamethyltrisiloxane or a similar organosiloxane oligomer.

A radical initiator may be any compound known in the art for standard radical polymerization reactions. The specific examples of such radical initiators are 2,2'-azobis(isobutyronitrile), 2,2'-azobis(2-methylbutyronitrile), 2,2'-azobis(2,4-dimethylvaleronitrile) or similar compounds of azobis type; benzoyl peroxide, lauroyl peroxide, tert-butyl peroxybenzoate, tert-butyl peroxy-2-ethylhexanoate or a similar organic peroxide. These radical initiators may be used alone or in a combination of two or more. The radical initiators may be used in an amount of from 0.1 to 5 parts by weight per 100 parts by weight of the components (A) and (B). A chain-transfer agent may be added. The chain-transfer agent may be 2-mercaptoethanol, butyl mercaptan, n-dodecyl mercaptan, 3-mercaptopropyltrimethoxysilane, a polydimethylsiloxane containing a mercaptopropyl group, or a similar compound of mercapto type; methylene chloride, chloroform, carbon tetrachloride, butyl bromide, 3-chloropropyltrimethoxysilane or a similar halogenated compound.

In the production of the polymer of vinyl type, after polymerization, the residual vinyl monomer that has not reacted may be removed under vacuum heating conditions.

To facilitate the preparation of the mixture of the starting material of cosmetic products, the number-average molecular mass of the vinyl polymer containing a carbosiloxane dendrimer may be selected within the range of between 3,000 and 2,000,000, such as between 5,000 and 800,000. It may be a liquid, a gum, a paste, a solid, a powder or any other form. The preferred forms are the solutions consisting of the dilution, in solvents, of a dispersion or of a powder.

The vinyl polymer may be a dispersion of a polymer of vinyl type having a carbosiloxane dendrimer structure in its side molecular chain, in a liquid such as a silicone oil, an organic oil, an alcohol or water.

The silicone oil may be a dimethylpolysiloxane with the two molecular ends capped with trimethylsiloxy groups, a copolymer of methylphenylsiloxane and of dimethylsiloxane having the two molecular ends capped with trimethylsiloxy groups, a copolymer of methyl-3,3,3-trifluoropropylsiloxane and of dimethylsiloxane having the two molecular ends capped with trimethylsiloxy groups, or similar nonreactive linear silicone oils, and also hexamethylcyclotrisiloxane, octamethylcyclotetrasiloxane, decamethylcyclo-pentasiloxane, dodecamethylcyclohexasiloxane, or a similar cyclic compound. In addition to the nonreactive silicone oils, modified polysiloxanes containing functional groups such as silanol groups, amino groups and polyether groups on the ends or within the side molecular chains may be used.

The organic oils may be isododecane, liquid paraffin, isoparaffin, hexyl laurate, isopropyl myristate, myristyl myristate, cetyl myristate, 2-octyldodecyl myristate; isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cocoa oil, jojoba oil, gum oil, sunflower oil, soybean oil, camelia oil, squalane, castor oil, cotton seed oil, coconut oil, egg yolk oil, propylene glycol monooleate, neopentyl glycol 2-ethylhexanoate, or a similar glycol ester oil; triglyceryl isostearate, the triglyceride of a fatty acid of coconut oil, or a similar oil of a polyhydric alcohol ester; polyoxyethylene lauryl ether, polyoxypropylene cetyl ether or a similar polyoxyalkylene ether.

The alcohol may be of any type that is suitable for use in combination with a cosmetic product starting material. For example, it may be methanol, ethanol, butanol, isopropanol or similar lower alcohols.

A solution or a dispersion of the alcohol should have a viscosity in the range from 10 to 10⁹ mPa at 25°C. To improve the sensory use properties in a cosmetic product, the viscosity may be within the range from 100 to 5 x 10⁸ mPa.s.

The solutions and the dispersions may be readily prepared by mixing a vinyl polymer having at least one carbosiloxane dendrimer-derived unit with a silicone oil, and organic oil, an alcohol or water. The liquids may be present in the step of polymerization of a vinyl polymer having at least one carbosiloxane dendrimer-derived unit. In this case, the residual vinyl monomer that has not reacted should be completely removed by heat treatment of the solution or dispersion under atmospheric pressure or reduced pressure.

In the case of a dispersion, the dispersity of the polymer of vinyl type may be improved by adding a surfactant.

Such a surfactant may be hexylbenzenesulfonic acid, octylbenzenesulfonic acid, decylbenzenesulfonic acid, dodecylbenzenesulfonic acid, cetylbenzenesulfonic acid, myristylbenzenesulfonic acid or anionic surfactants such as sodium salts of these acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, tallow-trimethylammonium hydroxide, coconut oil-trimethylammonium hydroxide, or a similar cationic surfactant; a polyoxyalkylene alkyl ether, a polyoxyalkylenealkylphenol, a polyoxyalkylene alkyl ester, the sorbitol ester of polyoxyalkylene, polyethylene glycol, polypropylene glycol, an ethylene oxide additive of diethylene glycol trimethylnonanol, and nonionic surfactants of the polyester type, and also mixtures.

In the dispersion, a mean particle diameter of the polymer of the vinyl type may be within a range of between 0.001 and 100 microns, and preferably between 0.01 and 50 microns. Indeed, outside the recommended range, a cosmetic product mixed with the emulsion will not have a nice enough feel on the skin or to the touch, or sufficient spreading properties or a pleasant sensation.

A vinyl polymer contained in the dispersion or the solution may have a concentration in a range of between 0.1% and 95% by weight, such as between 5% and 85% by weight. However, to facilitate the handling and the preparation of the mixture, the range may be between 10% and 75% by weight.

A vinyl polymer suitable for the present invention may also be one of the polymers described in the examples of application EP 0 963 751.

According to a preferred embodiment, a vinyl polymer grafted with a carbosiloxane dendrimer may be derived from the polymerization of:
(A1) 0.1 to 99 part(s) by weight of one or more acrylate or methacrylate monomer(s); and
(B1) 100 to 0.1 part(s) by weight of an acrylate or methacrylate monomer of a tri[tri(trimethylsiloxy) silylethyl dimethylsiloxyl] silylpropyl carbosiloxane dendrimer.

Monomers (A1) and (B1) correspond respectively to specific monomers (A) and (B).

According to an embodiment, a vinyl polymer having at least one carbosiloxane dendrimer-derived unit may include a unit derived from a tri[tri(trimethylsiloxy) silylethyldimethylsiloxy] silylpropyl carbosiloxane dendrimer corresponding to one of the formulas:

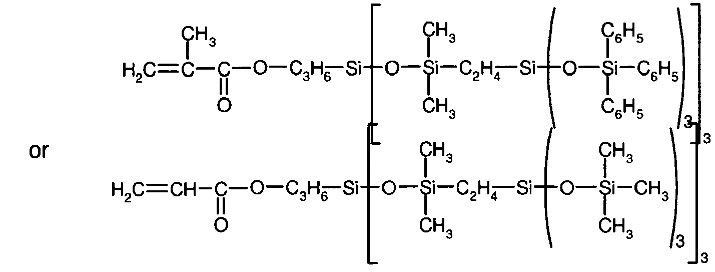

According to a preferred embodiment, a vinyl polymer having at least one carbosiloxane dendrimer-derived unit, used in the invention, comprises at least one butyl acrylate monomer.

According to an embodiment, a vinyl polymer may also include at least one fluorinated organic group.

Structures in which the polymerized vinyl units constitute the backbone and in which carbosiloxane dendritic structures as well as fluorinated organic groups are attached to side chains are particularly preferred.

The fluorinated organic groups can be obtained by replacing, with fluorine atoms, all or some of the hydrogen atoms of methyl, ethyl, propyl, isopropyl, butyl, isobutyl, tert-butyl, pentyl, neopentyl, hexyl, cyclohexyl, heptyl, octyl, nonyl, decyl, undecyl, dodecyl, tridecyl, tetradecyl, hexadecyl and octadecyl groups and other alkyl groups having from 1 to 20 carbon atoms, and also alkyloxyalkylene groups having from 6 to 22 carbon atoms.

The groups represented by the formula -(CH₂)ₓ-(CF₂)_{y}-R¹³ are suggested by way of examples of fluoroalkyl groups obtained by substituting fluorine atoms for hydrogen atoms of alkyl groups. In the formula, the index "x" is 0, 1, 2 or 3 and "y" is an integer from 1 to 20. R¹³ is an atom or a group selected from a hydrogen atom, a fluorine atom, -CH(CF₃)₂- or CF(CF₃)₂. Such fluorine-substituted alkyl groups are exemplified by linear or branched polyfluoroalkyl or perfluoroalkyl groups represented by the formulas given below: -CF₃, -C₂F₅, -nC₃F₇, -CF(CF₃)₂, -nC₄F₉, CF₂CF(CF₃)₂, -nC₅F₁₁, -nC₆F₁₃, -nC₈F₁₇, CH₂CF₃, -(CH(CF₃)₂, CH₂CH(CF₃)₂-CH₂(CF₂)₂F, -CH₂(CF₂)₃F, -CH₂(CF₂)₄F, -CH₂(CF₂)₆F, CH₂(CF₂)₈F, -CH₂CH₂CF₃, -CH₂CH₂(CF₂)₂F, -CH₂CH₂(CF₂)₃F, -CH₂CH₂(CF₂)₄F, -CH₂CH₂(CF₂)₆F, -CH₂CH₂(CF₂)₈F, -CH₂CH₂(CF₂)₁₀F, -CH₂CH₂(CF₂)₁₂F, CH₂CH₂(CF₂)₁₄F, -CH₂CH₂(CF₂)₁₆F, -CH₂CH₂CH₂CF₃, -CH₂CH₂CH₂(CF₂)₂F, -CH₂CH₂CH₂(CF₂)₂H, -CH₂(CF₂)₄H et -CH₂CH₂(CF₂)₃H.

The groups represented by -CH₂CH₂-(CF₂)ₘ-CFR¹⁴-[OCF₂CF(CF₃)]ₙ-OC₃F₇ are suggested as fluoroalkyloxyfluoroalkylene groups obtained by substituting fluorine atoms for hydrogen atoms of alkyloxyalkylene groups. In the formula, the index "m" is 0 or 1, "n" is 0, 1, 2, 3, 4 or 5, and R¹⁴ is a fluorine atom or CF₃. Such fluoroalkyloxyfluoroalkylene groups are exemplified by the perfluoroalkyloxyfluoroalkylene groups represented by the formulas given below: -CH₂CH₂CF(CF₃)-[OCF₂CF(CF₃)]ₙ-OC₃F₇, -CH₂CH₂CF₂CF₂-[OCF₂CF(CF₃)]ₙ-OC₃F₇.

The number-average molecular weight of the vinyl polymer used in the present invention may be between 3,000 and 2,000,000, such as between 5,000 and 800,000.

This type of fluorinated vinyl polymer may be obtained by addition:
- of a vinyl monomer (M2) without a fluorinated organic group,
- on a vinyl monomer (M1) containing fluorinated organic groups, and
- a carbosiloxane dendrimer (B) as defined above, with the general formula (I) as defined above,
by subjecting them to copolymerization.

Thus, according to an embodiment, a composition of the invention may include a vinyl polymer having at least one carbosiloxane dendrimer-derived unit and which is derived from the copolymerization of a vinyl monomer (M1) as defined above, optionally a vinyl monomer (M2) as defined above, and a carbosiloxane dendrimer (B) as defined above,
with said vinyl polymer having a copolymerization ratio between the monomer (M1) and the monomer (M2) of 0.1 to 100:99.9 to 0% by weight, and a copolymerization ratio between the sum of monomers (M1) and (M2) and monomer (B) of 0.1 to 99.9:99.9 to 0.1 % by weight.

The vinyl monomers (M1) containing fluorinated organic groups in the molecule are preferably monomers represented by the general formula:

(CH²)=CR¹⁵COOR^{f}.

In this formula, R¹⁵ is a hydrogen atom or a methyl group, R^{f} is a fluorinated organic group exemplified by the fluoroalkyl and fluoroalkyloxyfluoroalkylene groups described above. The compounds represented by the formulas given below are suggested by way of specific examples of the component (M1). In the formulas given below, "z" is an integer from 1 to 4.
CH₂=CCH₃COO-CF₃, CH₂=CCH₃COO-C₂F₅, CH₂=CCH3COO-nC₃F₇, CH₂=CCH₃COO-CF(CF₃)₂, CH₂=CCH₃COO-nC₄F₉, CH₂=CCH₃COO-CF(CF₃)₂, CH₂=CCH₃COO-nC₅F₁₁, CH₂=CCH₃COO-nC₆F₁₃, CH₂=CCH₃COO-nC₈F₁₇, CH₂=CCH₃COO-CH₂CF₃, CH₂=CCH₃COO-CH(CF₃)₂, CH₂=CCH₃COO-CH₂CH(CF₃)₂, CH₂=CCH₃COO-CH₂(CF₂)₂F, CH₂=CCH₃COO-CH₂(CF₂)₂F, CH₂=CCH₃COO-CH₂(CF₂)₄F, CH₂=CCH₃COO-CH₂(CF₂)₆F, CH₂=CCH₃COO-CH₂(CF₂)₈F, CH₂=CCH₃COO-CH₂CH₂CF₃, CH₂=CCH₃COO-CH₂CH₂(CF₂)₂F, CH₂=CCH₃COO-CH₂CH₂(CF₂)₃F, CH₂=CCH₃COO-CH₂CH₂(CF₂)₄F, CH₂=CCH₃COO-CH₂CH₂(CF₂)₆F, CH₂=CCH₃COO-CH₂CH₂(CF₂)₈F, CH₂=CCH₃COO-CH₂CH₂(CF₂)₁₀F, CH₂=CCH₃COO-CH₂CH₂(CF₂)₁₂F, CH₂=CCH₃COO-CH₂CH₂(CF₂)₁₄F, CH₂=CCH₃COO-CH₂-CH₂-(CF₂)₁₆F, CH₂=CCH₃COO-CH₂CH₂CH₂CF₃, CH₂=CCH₃COO-CH₂CH₂CH₂(CF₂)₂F, CH₂=CCH₃COO-CH₂CH₂CH₂(CF₂)₂H, CH₂=CCH₃COO-CH₂ (CF₂)₄H, CH₂=CCH₃COO-(CF₂)₃H, CH₂=CCH₃COO-CH₂CH₂CF(CF₃)-[OCF₂-CF(CF₃)]z-OC₃F₇, CH₂=CCH₃COO-CH₂CH₂CF₂CF₂-[OCF₂-CF(CF₃)]z-OC₃F₇, CH₂=CHCOO-CF₃, CH2=CHCOO-C₂F₅, CH₂=CHCOO-nC₃F₇, CH₂=CHCOO-CF(CF₃)₂, CH₂=CHCOO-nC₄F₉, CH₂=CHCOO-CF₂CF(CF₃)₂, CH₂=CHCOO-nC₅F₁₁, CH₂=CHCOO-nC₆F₁₃, CH₂=CHCOO-nC₈F₁₇, CH₂=CHCOO-CH₂CF₃, CH₂=CHCOO-CH(CF₃)₂, CH₂=CHCOO-CH₂CH(CF₃)₂, CH₂=CHCOO-CH₂(CF₂)₂F, CH₂=CHCOO-CH₂(CF₂)₃F, CH₂=CHCOO-CH₂(CF₂)₄F, CH₂=CHCOO-CH₂(CF₂)₆F, CH₂=CHCOO-CH₂(CF₂)₈F, CH₂=CHCOO-CH₂CH₂CF₃, CH₂=CHCOO-CH₂CH₂ (CF₂)₂F, CH₂=CHCOO-CH₂CH₂(CF₂)₃F, CH₂=CHCOO-CH₂CH₂(CF₂)₄F, CH₂=CHCOO-CH₂CH₂(CF₂)₆F, CH₂=CHCOO-CH₂CH₂(CF₂)₈F, CH₂=HCOO-CH₂CH₂(CF₂)₁₀F, CH₂-CHCOO-CH₂CH₂-(CF₂)₁₂F, CH₂=CHCOO-CH₂CH₂(CF₂)₁₄F, CH₂=CHCOO-CH₂CH₂(CF₂)₁₆F, CH₂=CHCOO-CH₂CH₂CH₂CF₃, CH₂=CHCOO-CH₂CH₂CH₂(CF₂)₂F, CH₂=CHCOO-CH₂CH₂CH₂(CF)₂H, CH₂=CHCOO-CH₂(CF₂)₄H, CH₂=CHCOO-CH₂CH₂(CF₂)₃H, CH₂=CHCOO-CH₂CH₂CF(CF₃)-, [OCF₂-CF(CF₃)]_{z}-OC₃F₇, CH₂=CHCOO-CH₂CH₂CF₂CF₂(CF₃)-[OCF₂-CF(CF₃)]₂-OC₃F₇.

Among these, the vinyl polymers represented by the formulas presented below are preferred:
CH₂=CHCOO-CH₂CH₂(CF₂)₆F, CH₂=CHCOO-CH₂CH₂(CF₂)₈F, CH₂=CCH₃COO-CH₂CH₂(CF₂)₆F, CH₂=CCH₃COO-CH₂CH₂(CF₂)₈F, CH₂=CHCOO-CH₂CF₃, CH₂= CCH₃COO-CH₂CF₃.

The vinyl polymers represented by the formulas presented below are especially preferred:
CH₂=CHCOO-CH₂CF₃, CH₂=CCHCOO-CH₂CF₃.

The vinyl monomers (M2) not containing any fluorinated organic groups in the molecule can be any monomers containing radical-polymerizable vinyl groups that are exemplified, for example, by methyl acrylate, methyl methacrylate, ethyl acrylate, ethyl methacrylate, n-propyl acrylate, n-propyl methacrylate, isopropyl acrylate, isopropyl methacrylate, or other lower alkyl acrylates or methacrylates; glycidyl acrylate, glycidyl methacrylate; n-butyl acrylate, n-butyl methacrylate, isobutyl acrylate, isobutyl methacrylate, tert-butyl acrylate, tert-butyl methacrylate, n-hexyl acrylate, n-hexyl methacrylate, n-hexyl acrylate, n-hexyl methacrylate, cyclohexyl acrylate, cyclohexyl methacrylate, 2-ethylhexyl acrylate, 2-ethylhexyl methacrylate, octyl acrylate, octyl methacrylate, lauryl acrylate, lauryl methacrylate, stearyl acrylate, stearyl methacrylate, or other higher acrylates and methacrylates; vinyl acetate, vinyl propionate, or other lower fatty acid vinyl esters; vinyl butyrate, vinyl caproate, vinyl 2-ethylhexanoate, vinyl laurate, vinyl stearate, or other higher fatty acid esters; styrene, vinyltoluene, benzyl acrylate, benzyl methacrylate, phenoxyethyl acrylate, phenoxyethyl methacrylate, vinylpyrrolidone, and other vinylaromatic monomers; dimethylaminoethyl acrylate, dimethylaminoethyl methacrylate, diethylaminoethyl acrylate, diethylaminoethyl methacrylate, and other aminovinyl monomers, acrylamide, methacrylamide, N-methylolacrylamide, N-methylolmethacrylamide, N-methoxymethylacrylamide, N-methoxymethylmethacrylamide, isobutoxymethoxyacrylamide, isobutoxymethoxymethacrylamide, N,N-dimethylacrylamide, N,N-dimethylmethacrylamide, and other vinylamide monomers; hydroxyethyl acrylate, hydroxyethyl methacrylate, acrylic acid hydroxypropyl alcohol, methacrylic acid hydroxypropyl alcohol, and other hydroxy vinyl monomers; acrylic acid, methacrylic acid, itaconic acid, crotonic acid, fumaric acid, maleic acid, and other vinylcarboxylic acid monomers; tetrahydrofurfuryl acrylic, tetrahydrofurfuryl methacrylate, butoxyethyl acrylate, butoxyethyl methacrylate, ethoxydiethylene glycol acrylate, ethoxydiethylene glycol methacrylate, polyethylene glycol acrylate, polyethylene glycol methacrylate, polypropylene glycol monoacrylate, polypropylene glycol monomethacrylate, hydroxybutyl vinyl ether, cetyl vinyl ether, 2-ethylhexyl vinyl ether and other vinyl monomers containing an ether bond; acryloxypropyltrimethoxysilane, methacryloxypropyltrimethoxysilane, polydimethyl-siloxanes containing acryl or methacryl groups at one of the ends, polydimethylsiloxanes containing alkenylaryl groups at one of the ends and other silicone compounds containing unsaturated groups; butadiene; vinyl chloride; vinylidene chloride, acrylonitrile, methacrylonitrile; dibutyl fumarate; maleic anhydride; dodecylsuccinic anhydride; acryl glycidyl ether, methacryl glycidyl ether, 3,4-epoxycyclohexylmethyl acrylate, 3,4-epoxycyclohexylmethyl methacrylate, alkali metal salts, ammonium salts and organic amine salts of acrylic acid, of methacrylic acid, of itaconic acid, of crotonic acid, of fumaric acid, of maleic acid and of other radical-polymerizable unsaturated carboxylic acids, radical-polymerizable unsaturated monomers containing sulfonic acid groups, such as styrene sulfonic acid and also the alkali metal salts thereof, the ammonium salts thereof and the organic amine salts thereof; the quaternary ammonium salts derived from acrylic acid or from methacrylic acid, such as 2-hydroxy-3-methacryloxypropyltrimethylammonium chloride, methacrylic acid esters of a tertiary amine alcohol, such as the diethylamine ester of methacrylic acid and quaternary ammonium salts thereof.

In addition, it is also possible to use, by way of vinyl monomers (M2), the polyfunctional vinyl monomers which are exemplified, for example, by trimethylolpropane triacrylate, trimethylolpropane trimethacrylate, pentaerythritol triacrylate, pentaerythritol trimethacrylate, ethylene glycol diacrylate, ethylene glycol dimethacrylate, tetraethylene glycol diacrylate, tetraethylene glycol dimethacrylate, polyethylene glycol diacrylate, polyethylene glycol dimethacrylate, 1,4-butanediol diacrylate, 1,4-butanediol dimethacrylate, 1,6-hexanediol diacrylate, 1,6-hexanediol dimethacrylate, neopentyl glycol diacrylate, neopentyl glycol dimethacrylate, trimethylolpropanetrioxyethyl acrylate, trimethylolpropanetrioxyethyl methacrylate, tris(2-hydroxyethyl)isocyanurate diacrylate, tris(2-hydroxyethyl)isocyanurate dimethacrylate, tris(2-hydroxyethyl)isocyanurate triacrylate, tris(2-hydroxyethyl)isocyanurate trimethacrylate, polydimethylsiloxane in which the two ends of the molecular chain are blocked with alkenylaryl groups, and other silicone compounds containing unsaturated groups.

As regards the ratio mentioned above in which (M1) and (M2) are copolymerized, the weight ratio between (M1) and (M2) is preferably within the range from 1:99 to 100:0.

Y may be selected, for example, from organic groups containing acrylic or methacrylic groups, organic groups containing an alkenylaryl group, or alkenyl groups having from 2 to 10 carbon atoms.

The organic groups containing acrylic or methacrylic groups and the alkenylaryl group are as defined above.

Among the compounds (B), the following compounds may, for example, be mentioned:

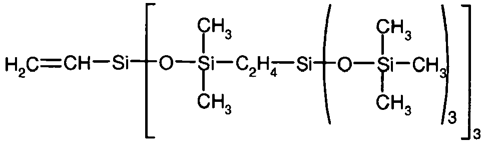

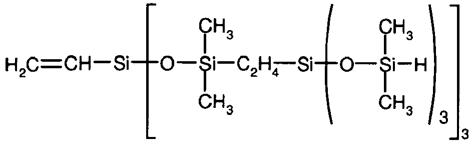

The carbosiloxane dendrimers (B) may be prepared using the process for preparing the siloxane/silylalkylene branched copolymers described in document EP 1 055 674.

For example, they may be prepared by subjecting organic alkenyl silicone compounds and silicone compounds including hydrogen atoms bonded to the silicon, represented by formula (IV) as defined above, to a hydrosilylation reaction.

The copolymerization ratio (by weight) between monomer (B) and monomers (M1) and (M2) is preferably within the range from 1:99 to 99:1, and even more preferably within the range from 5:95 to 95:5.

Amino groups may be introduced into the side chains of the vinyl polymer using, included in the component (M2), vinyl monomers containing amino groups, such as dimethylaminoethyl acrylate, dimethylaminoethyl methacrylate, diethylaminoethyl acrylate and diethylaminoethyl methacrylate, followed by performing a modification with potassium acetate monochloride, ammonium acetate monochloride, the aminomethylpropanol salt of monochloroacetic acid, the triethanolamine salt of monobromoacetic acid, sodium monochloropropionate, and other alkali metal salts of halogenated fatty acids; otherwise, carboxylic acid groups may be introduced into the side chains of the vinyl polymer using, included in the component (M2), vinyl monomers containing carboxylic acids, such as acrylic acid, methacrylic acid, itaconic acid, crotonic acid, fumaric acid and maleic acid, and the like, followed by neutralizing the product with triethylamine, diethylamine, triethanolamine and other amines.

A fluorinated vinyl polymer may be one of the polymers described in the examples of application WO 03/045337.

According to a preferred embodiment, a grafted vinyl polymer for the purpose of the present invention may be carried in an oil or a mixture of oil(s), such as volatile oil(s), in particular selected from silicone oils and hydrocarbon oils and mixtures thereof.

According to a particular embodiment, a silicone oil suitable for the invention may be cyclopentasiloxane.

According to a particular embodiment, a hydrocarbon oil suitable for the invention may be isododecane.

The vinyl polymers grafted with at least one carbosiloxane dendrimer-derived unit that may be particularly suitable for the present disclosure are the polymers sold under the names TIB 4-100, TIB 4-101, TIB 4-120, TIB 4-130, TIB 4-200, FA 4002 ID (TIB 4-202), TIB 4-220, FA 4001 CM (TIB 4-230) by the Dow Corning company.

According to an embodiment, the composition according to the present invention includes the vinyl polymer having at least one carbosiloxane dendrimer-derived unit in a content with respect to active material ranging from 0.5% to 20% by weight, in particular from 1% to 15%, more specifically from 1.5% to 10% and preferably from 3% to 5% by weight with respect to the total weight of said composition.

### Monoalcohol

The compositions of the invention include at least one monoalcohol comprising 2 to 8 carbon atoms, in particular 2 to 6 carbon atoms and more particularly 2 to 4 carbon atoms.

The compositions of the invention may include one or more monoalcohol(s).

This monoalcohol may be represented, for example, by the formula RₐOH, in which Rₐ represents a linear or branched alkyl group containing 2 to 8 carbon atoms.

As examples of monoalcohols, ethanol, isopropanol, propanol and butanol may be cited.

According to an embodiment, the compositions of the invention include ethanol.

The amount of monoalcohol(s) ranges from 10% to 40% by weight in the composition, preferably from 10% to 20% by weight and even more preferably from 10% to 15% by weight with respect to the total weight of said composition.

The amounts by weight of monoalcohol(s) correspond either to the amount by weight of the monoalcohol if the composition contains only one monoalcohol or to the total amount by weight of all of the monoalcohols if the composition includes a mixture of a plurality of monoalcohols.

According to an embodiment, the compositions of the invention include 11 % to 15%, preferably 11.5% to 15%, by weight of monoalcohol(s), preferably ethanol, with respect to the total weight of said composition.

### Physiologically acceptable medium

In addition to the compounds indicated above, a composition according to the invention includes a physiologically acceptable medium.

The term "physiologically acceptable medium" is intended to denote a medium that is particularly suitable for the application of a composition of the invention to the skin or the lips.

The physiologically acceptable medium is generally suitable for the nature of the support to which the composition should be applied, and also for the way in which the composition should be packaged.

A composition of the invention may be a dispersion or an emulsion.

A dispersion may be prepared in an aqueous phase or in an oily phase.

The compositions of the invention may be anhydrous or in the form of a W/O emulsion, an O/W emulsion or even a multiple emulsion. An emulsion may have an oily or aqueous continuous phase. Such an emulsion may, for example, be an inverse emulsion (W/O) or a direct emulsion (O/W), or else a multiple emulsion (W/O/W or O/W/O). In the case of emulsions, inverse emulsions (W/O) are preferable.

According to an embodiment, the amount of water in the compositions of the invention is less than 30% by weight, preferably less than 20% by weight and more preferably less than 10% by weight, and even more preferably less than 5% by weight with respect to the total weight of the composition.

According to a particular embodiment, the composition of the invention is anhydrous or includes less than 5% by weight, more preferably less than 2% by weight, and even more preferably less than 1% by weight, with respect to the total weight of said composition.

According to an embodiment, the compositions according to the invention include an alcohol or hydroalcohol phase, and a fatty phase, in which said alcohol or hydroalcohol phase contains the monoalcohol as defined above and said fatty phase contains the vinyl polymer having at least one carbosiloxane dendrimer-derived unit as defined above.

### Alcoholic or hydro-alcoholic phase

The composition according to the invention includes an alcoholic phase including at least one monoalcohol as defined above.

The alcoholic phase may include water (hydro-alcoholic phase). A water suitable for the invention may be a floral water such as cornflower water and/or a mineral water such as Vittel water, Lucas water or La Roche Posay water and/or a spring water.

According to an embodiment, the amount of water in the compositions of the invention is less than 30% by weight, preferably less than 20% by weight and more preferably less than 10% by weight and even more preferably less than 5% by weight with respect to the total weight of the composition.

According to a particular embodiment, the composition according to the invention is anhydrous or contains less than 5% by weight of water, more preferably less than 2% by weight of water, even more preferably less than 1% by weight of water, with respect to the total weight of said composition.

In addition to the lower monoalcohol(s) defined above, this alcoholic or hydro-alcoholic phase may contain other alcohol(s), in particular polyethylene glycols having 6 to 80 ethylene oxide units; polyols such as propylene glycol, isoprene glycol, butylene glycol, glycerine, sorbitol, glycols such as propylene glycol, butylene glycol, dipropylene glycol, diethylene glycol, glycol ethers such as mono-, di- or triethylene glycol alkyl(C₁-C₄)ether and mixtures thereof.

The alcoholic or hydro-alcoholic phase may also include stabilizers, such as sodium chloride, magnesium dichloride and magnesium sulfate.

The alcoholic or hydro-alcoholic phase may also include any water-soluble or water-dispersible compound compatible with an aqueous phase, such as gelling agents, film-forming polymers, thickeners, surfactants and mixtures thereof.

### Fatty phase

A cosmetic composition according to the present invention may include at least one liquid and/or solid fatty phase.

According to an embodiment of the present invention, the vinyl polymer having at least one carbosiloxane dendrimer-derived unit is present in the fatty phase.

In particular, a composition of the invention may include at least one liquid fatty phase, in particular at least one oil as mentioned below.

The term "oil" is intended to mean any fatty substance in liquid form at ambient temperature (20-25°C) and at atmospheric pressure. These oils may be of animal, plant, mineral or synthetic origin.

According to an embodiment, the fatty phase of the compositions of the invention includes at least one volatile oil and/or at least one non-volatile oil.

### Volatile oils

According to an embodiment, the fatty phase of the compositions of the invention includes at least one volatile oil. The fatty phase of the compositions of the invention may include a mixture of a plurality of volatile oils.

The term "volatile oil" is intended to mean any non-aqueous medium capable of evaporating on contact with the skin or the lips, in less than one hour, at ambient temperature and at atmospheric pressure. The volatile oil is a volatile cosmetic oil that is liquid at ambient temperature. More specifically, a volatile oil has an evaporation rate of between 0.01 and 200 mg/cm²/mn, inclusive.

To measure this evaporation rate, 15g of oil or an oil mixture to be tested are introduced into a crystallizer with a diameter of 7cm, placed on a scale located in a large chamber of around 0.3m³, with controlled temperature, at 25°C, and hygrometry, at 50% relative humidity. The liquid is left to evaporate freely, without being agitated, by allowing ventilation with a fan (PAPST-MOTOREN, reference 8550 N, rotating at 2700 rpm) arranged vertically above the crystallizer containing said oil or said mixture, with the blades being directed toward the crystallizer and at a distance of 20cm with respect to the crystallizer base. The mass of oil remaining in the crystallizer is measured at regular intervals. The evaporation rates are expressed in mg of oil evaporated per unit of surface (cm²) and per unit of time (minutes).

The volatile oils may be hydrocarbon-, silicone- or fluorine-based.

The term "silicone oil" is intended to mean an oil comprising at least one silicon atom, and in particular at least one SiO group.

The term "fluorine oil" is intended to mean an oil comprising at least one fluorine atom.

The term "hydrocarbon oil" is intended to mean an oil containing mainly hydrogen and carbon atoms.

The oils may optionally comprise oxygen, nitrogen, sulfur and/or phosphorus atoms, for example in the form of hydroxyl or acid radicals

The volatile oils may be selected from hydrocarbon oils having from 8 to 16 carbon atoms, and in particular branched C₈-C₁₆ alkanes (also known as isoparaffins or isoalkanes), such as isododecane (also known as 2,2,4,4,6-pentamethylheptane), isodecane or isohexadecane, and, for example, the oils sold under the trade names ISOPARS^{®} or PERMETHYLS^{®}.

It is also possible to cite, as a hydrocarbon volatile oil, linear C₉-C₁₇ alkanes, such as dodecane (C₁₂) and tetradecane (C₁₄), sold respectively under the names PARAFOL^{®} 12-97 and PARAFOL^{®} 14-97 (Sasol), and, as alkanes obtained according to the method described in the international application WO 2007/068371 A1, such as the undecane (C₁₁) and tridecane (C₁₃) mixture sold under the name CETIOL^{®} UT (Cognis).

Among the volatile hydrocarbon oils, isododecane and the undecane (C₁₁) and tridecane (C₁₃) mixture are preferred.

It is also possible to use, as volatile oils, volatile silicones, such as, for example, volatile linear or cyclic silicones, in particular those having a viscosity below or equal to 8 centistokes (cSt) (8 x 10⁻⁶m²/s), and having, in particular, from 2 to 10 silicon atoms, and in particular from 2 to 7 silicon atoms, in which these silicones optionally comprise alkyl or alkoxyl groups having from 1 to 10 carbon atoms. It is possible to cite, as a volatile silicone oil that can be used in the invention, in particular, dimethicones with a viscosity of 5 and 6 cSt, octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane, dodecamethyl pentasiloxane, and mixtures thereof.

More specifically, as a volatile silicone oil, it is possible to cite linear or cyclic silicone oils having from 2 to 7 silicon atoms, in which these silicones optionally comprise alkyl or alkoxyl groups having from 1 to 10 carbon atoms.

As preferred examples, it is possible to cite decamethyl cyclopentasiloxane, dodecamethyl cyclohexasiloxane and dodecamethyl pentasiloxane.

Among the volatile silicone oils, dodecamethyl pentasiloxane is preferred.

It is possible to cite, as a volatile fluorine oil, for example, nonafluoromethoxybutane or perfluoromethylcyclopentane, and mixtures thereof.

According to an embodiment, the fatty phase of the compositions of the invention includes 40% to 100% by weight, preferably 60% to 98% by weight, and preferably 80% to 95% by weight of volatile oil(s) with respect to the total weight of the fatty phase.

### Non-volatile oils

According to an embodiment, the fatty phase of the compositions of the invention includes at least one non-volatile oil. The fatty phase of the compositions of the invention may include a mixture of a plurality of non-volatile oils.

The term "non-volatile oil" is intended to mean an oil remaining on the skin or keratin fiber at ambient temperature and atmospheric pressure. More specifically, a non-volatile oil has an evaporation rate strictly below 0.01 mg/cm²/min.

The non-volatile oils may, in particular, be chosen from among the non-volatile hydrocarbon, fluorine and/or silicone oils.

It is possible to cite, as a non-volatile hydrocarbon oil:
- hydrocarbon oils of animal origin, such as perhydrosqualene,
- hydrocarbon oils of plant origin, such as phytostearyl esters, for instance phytostearyl oleate, phytostearyl isostearate and lauroyl/octyldodecyl/phytostearyl glutamate (AJINOMOTO, ELDEW PS203), triglycerides constituted of fatty acid esters of glycerol, in particular in which the fatty acids may have chain lengths ranging from C₄ to C₃₆, and in particular from C₁₈ à C₃₆, it being possible for these oils to be linear or branched, and saturated or unsaturated; these oils may in particular be heptanoic or octanoic triglycerides, shea oil, alfalfa oil, poppy seed oil, pumpkin oil, millet oil, barley oil, quinoa oil, rye oil, candlenut oil, passionflower oil, shea butter, aloe oil, sweet almond oil, peach kernel oil, groundnut oil, argan oil, avocado oil, baobab oil, barrage oil, broccoli oil, calendula oil, camelina oil, canola oil, carrot oil, safflower oil, hemp oil, rapeseed oil, cotton seed oil, coconut oil, marrow seed oil, wheat germ oil, jojoba oil, lily oil, macadamia oil, corn oil, meadowfoam oil, St. John's Wort oil, monoi oil, hazelnut oil, apricot kernel oil, nut oil, olive oil, evening primrose oil, palm oil, blackcurrant seed oil, kiwi seed oil, grape seed oil, pistachio oil, pumpkin oil, winter squash oil, quinoa oil, musk rose oil, sesame oil, soya oil, sunflower oil, castor oil and watermelon oil, and mixtures thereof, or alternatively caprylic/capric acid triglycerides, for instance those sold by the STEARINERIES DUBOIS company or those sold under the names MIGLYOL 810^{®}, 812^{®} and 818^{®} by the DYNAMIT NOBEL company;
- linear or branched hydrocarbons of mineral or synthetic origin, such as liquid paraffins and derivatives thereof, petroleum jelly, polydecenes, polybutenes, hydrogenated polyisobutene such as Parleam, squalane;
- synthetic ethers having from 10 to 40 carbon atoms;
- synthetic esters, for instance oils of formula R₁COOR₂, in which R₁ represents a linear or branched fatty acid residue containing from 1 to 40 carbon atoms, and R₂ represents a hydrocarbon-based chain, in particular a branched chain, containing from 1 to 40 carbon atoms provided that R₁ et R₂ is greater than or equal to 10. The esters may in particular be selected from fatty acid and alcohol esters, for instance: cetostearyl octanoate, isopropyl alcohol esters, such as isopropyl myristate, isopropyl palmitate, ethyl palmitate, 2-ethylhexyl palmitate, isopropyl stearate or isostearate, isostearyl isostearate, octyl stearate, hydroxylated esters, for instance isostearyl lactacte, oetyl hydroxystearate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate and palmitate, alkyl benzoate, polyethylene glycol diheptanoate, propylene glycol 2-diethylhexanoate, and mixtures thereof, C₁₂-C₁₅ alkyl benzoates, hexyl laurate, neopentanoic acid esters, for instance isodecyl neopentanoate, isotridecyl neopentanoate, isostearyl neopentanoate, or octyldodecyl neopentanoate, isononanoic acid esters, for instance isononyl isononanoate, isotridecyl isononanoate and octyl isononanoate, hydroxylated esters such as isostearyl lactate and diisostearyl malate
- polyol esters and pentaerythritol esters, for instance dipentaerythrityl tetrahydroxystearate/tetraisostearate,
- esters of diol dimers and diacid dimers, such as Lusplan DD-DA5^{®} and Lusplan DD-DA7^{®}, sold by the NIPPON FINE CHEMICAL company and described in the application US 2004-175338,
- copolymers of a diol dimer and of a diacid dimer and esters thereof, such as copolymers of dilinoleyl diol dimers/dilinoleic dimers and esters thereof, for instance Plandool-G,
- copolymers of polyols and of diacid dimers, and esters thereof, such as Hailuscent ISDA, or the copolymer of dilinoleic acid/butanediol,
- fatty alcohols that are liquid at ambient temperature, with a branched and/or unsaturated carbon chain having from 12 to 26 carbon atoms, for instance 2-octyldodecanol, isostearyl alcohol, oleyl alcohol, 2-hexyldecanol, 2-butyloctanol and 2-u ndecylpentadecanol,
- C₁₂-C₂₂, higher fatty acids, such as oleic acid, linoleic acid or linolenic acid, and mixtures thereof, and,
- dialkyl carbonates, the two alkyl chains possibly being identical or different, such as the dicaprylyl carbonate sold under the name CETIOL CC^{®}, by COGNIS,
- oils of higher molar mass having in particular a molar mass ranging from approximately 400 to approximately 10,000 g/mol, in particular from approximately 650 to approximately 10,000 g/mol, in particular from approximately 750 to approximately 7,500 g/mol, and more particularly ranging from approximately 1,000 to approximately 5,000 g/mol. As oils of higher molar mass that can be used in the invention, mention may in particular be made of the oils selected from:
   - lipophilic polymers,
   - linear fatty acid esters having a total carbon number ranging from 35 to 70,
   - hydroxylated esters,
   - aromatic esters,
   - esters of C₂₄-C₂₈ branched fatty acids or fatty alcohols,
   - silicone oils,
   - oils of plant origin,
   - and mixtures thereof;
- fluorine oils optionally partially hydrocarbon-based and/or silicone-based, such as fluorosilicone oils, fluorinated polyethers or fluorinated silicones, as described in document EP-A-847 752;
- silicone oils, such as polydimethylsiloxanes (PDMS) which are non-volatile and linear or cyclic; polydimethylsiloxanes comprising alkyl, alkoxy or phenyl groups which are pendant or at the end of the silicone chain, said groups having from 2 to 24 carbon atoms; phenylated silicones, such as phenyl trimethicones, phenyl dimethicones, phenyltrimethylsiloxydiphenylsiloxanes, diphenyl dimethicones, diphenylmethyldiphenyl-trisiloxanes or (2-phenylethyl)trimethylsiloxysilicates, and
- mixtures thereof.

Among the linear or branched hydrocarbons, of mineral or synthetic origin, paraffin oils or petroleum jelly are preferably used.

Among the hydrocarbon oils of plant origin, it is possible to cite, preferably, plant oils, such as sweet almond oil, jojoba oil or macadamia nut oil.

Among the synthetic oils such as synthetic esters, isodecyl neopentanoate or isononyl isononanoate is used in particular, and among the synthetic ethers, dicapryl ether is preferably used.

Among the non-volatile silicone oils, polydimethylsiloxanes, phenyltrimethicone or alkyldimethicones such as cetyl dimethicone are preferably used.

According to an embodiment, the fatty phase of the compositions of the invention does not include a non-volatile oil.

According to an embodiment, the fatty phase of the compositions of the invention includes less than 60% by weight, preferably from 1% to 40% by weight and preferably from 2% to 20% by weight of non-volatile oil(s) with respect to the total weight of the fatty phase.

According to an embodiment, the fatty phase of the compositions of the invention includes:
- from 40% to 100%, preferably from 60% to 98%, and more specifically from 80% to 95% by weight of volatile oil(s) with respect to the total weight of the fatty phase, and
- less than 60%, preferably from 1% to 40%, and more particularly from 2% to 20% by weight of non-volatile oil(s) with respect to the total weight of the fatty phase.

According to an embodiment, the fatty phase of the compositions of the invention represents a percentage ranging from 25% by 85%, preferably ranging from 40% to 75% and even more preferably ranging from 50% to 70% with respect to the total weight of the composition.

### Lipophilic structuring agent

A composition according to the invention may also comprise at least one agent for structuring a liquid fatty phase, selected from a wax, a pasty compound, and mixtures thereof.

In particular, a wax suitable for the invention may especially be selected from waxes of animal, plant, mineral and synthetic origin, and mixtures thereof.

By way of examples of waxes that can be used according to the invention, mention may be made of:
- waxes of animal origin, such as beeswax, spermaceti, lanolin wax and lanolin derivatives, plant waxes such as carnauba wax, candelilla wax, ouricury wax, Japan wax, cocoa butter, cork fiber wax or sugarcane wax,
- mineral waxes, for example paraffin wax, petroleum jelly wax, lignite wax or microcrystalline waxes or ozokerites,
- synthetic waxes, among which are polyethylene waxes, and waxes obtained by Fisher-Tropsch synthesis
- silicone waxes, in particular substituted linear polysiloxanes; mention may, for example, be made of silicone polyether waxes, alkyl dimethicones or alkoxy dimethicones having from 16 to 45 carbon atoms, alkyl methicones such as the C₃₀-C₄₅ alkyl methicone sold under the trade name "AMS C 30(R)" by Dow Corning,
- hydrogenated oils solid at 25°C such as hydrogenated castor oil, hydrogenated jojoba oil, hydrogenated palm oil, hydrogenated tallow or hydrogenated coconut oil, and fatty esters that are solid at 25°C, such as C₂₀-C₄₀ alkyl stearate sold under the trade name "KESTER WAX K82H" by the KOSTER KEUNEN company,
- and/or mixtures thereof.

Preferably, polyethylene waxes, microcrystalline waxes, carnauba waxes, hydrogenated jojoba oil, candelilla waxes, beeswaxes and/or mixtures thereof will be used.

A composition according to the invention may include at least one pasty compound.

The presence of a pasty compound may make it possible to advantageously confer improved comfort when a composition of the invention is deposited on keratin fibers.

Such a compound may advantageously be selected from lanolin and derivatives thereof; polymeric or non-polymeric silicone compounds; polymeric or non-polymeric fluorine compounds; vinyl polymers, in particular olefin homopolymers; olefin copolymers; hydrogenated diene homopolymers and copolymers; linear or branched and homo- or copolymeric oligomers of alkyl (meth)acrylates preferably having a C₈-C₃₀ alkyl group; homo- and copolymeric oligomers of vinyl esters having C₈-C₃₀ alkyl groups; homo- and copolymeric oligomers of vinyl ethers having C₈-C₃₀ alkyl groups; liposoluble polyethers resulting from polyetherification between one or more C₂-C₁₀₀, in particular C₂-C₅₀ diols; fatty acid or alcohol esters; and mixtures thereof.

Among the esters, it is possible to cite in particular:
- the esters of an oligomeric glycerol, especially the esters of diglycerol, for instance polyglyceryl-2 triisostearate, the condensates of adipic acid and of glycerol, for which a portion of the hydroxyl groups of the glycerols have reacted with a mixture of fatty acids, such as stearic acid, capric acid, stearic acid and isostearic acid and 12-hydroxystearic acid, such as, in particular, those sold under the trade mark Softisan 649 by the Sasol company, or such as bisdiglyceryl polyacyladipate-2; the arachidyl propionate sold under the trade mark Waxenol 801 by Alzo; phytosterol esters; triglycerides of fatty acids and derivatives thereof, such as hydrogenated cocoglycerides; non-cross-linked polyesters resulting from polycondensation between a linear or branched C₄-C₅₀ dicarboxylic acid or polycarboxylic acid and a C₂-C₅₀ diol or polyol; aliphatic esters of an ester resulting from the esterification of an aliphatic hydroxycarboxylic acid ester with an aliphatic carboxylic acid; polyesters resulting from the esterification, with a polycarboxylic acid, of an aliphatic hydroxycarboxylic acid ester, said ester comprising at least two hydroxyl groups, such as the products Risocast DA-H^{®}, and Risocast DA-L^{®}; and mixtures thereof.

The structuring agent(s) may be present in a composition of the invention in a content ranging from 0.1% to 30% by weight of agents, such as from 0.5% to 20% by weight, relative to the total weight of the composition.

### Thickeners

Depending on the fluidity of the composition that it is desired to obtain, one or more thickeners or gelling agents may be incorporated into a composition of the invention.

A thickener or gelling agent suitable for the invention may be hydrophilic, i.e. water-soluble or water-dispersible.

As hydrophilic gelling agents, mention may in particular be made of water-soluble or water-dispersible thickening polymers. Said polymers may in particular be selected from: modified or unmodified carboxyvinyl polymers, such as the products sold under the name Carbopol (CTFA name: carbomer) by the Goodrich company; polyacrylates and polymethacrylates, such as the products sold under the names Lubrajel and Norgel by the GUARDIAN company or under the name Hispagel by the HISPANO CHIMICA company; polyacrylamides; 2-acrylamido-2-methylpropanesulfonic acid polymers and copolymers, which are optionally cross-linked and/or neutralized, such as the poly(2-acrylamido-2-methylpropanesulfonic acid) sold by the CLARIANT company under the name "Hostacerin AMPS" (CTFA name: ammonium polyacryldimethyltauramide); cross-linked anionic acrylamide/AMPS copolymers, in the form of a W/O emulsion, such as those sold under the name SEPIGEL 305 (CTFA name: Polyacrylamide/C13-14 Isoparaffin/Laureth-7) and under the name SIMULGEL 600 (CTFA name: Acrylamide/Sodium acryloyldimethyltaurate copolymer/Isohexadecane/Polysorbate 80) by the SEPPIC company; polysaccharide biopolymers, such as xanthan gum, guar gum, carob gum, gum acacia, scleroglucans, chitin derivatives and chitosan derivatives, carrageenans, gellans, alginates, or celluloses such as microcrystalline cellulose, carboxymethylcellulose, hydroxymethylcellullose and hydroxypropylcellulose; and mixtures thereof.

A thickener or gelling agent suitable for the invention may be lipophilic, it may be inorganic or organic.

As lipophilic thickeners, mention may, for example, be made of modified clays, such as modified magnesium silicate (Bentone gel VS38 of RHEOX), modified hectorites such as hectorite modified with a C₁₀ to C₂₂ fatty acid ammonium chloride, for instance hectorite modified with distearyldimethylammonium chloride, for instance the product sold under the name Bentone 38V^{®} by the ELEMENTIS company or that sold under the name "Bentone 38 CE" by the RHEOX company or that sold under the name Bentone Gel V5 5V by the ELEMENTIS company.

The polymeric organic lipophilic gelling agents are, for example, partially or totally cross-linked elastomeric organopolysiloxanes with a three-dimensional structure, such as those sold under the names KSG6^{®}, KSG16^{®} and KSG18^{®} by the SHIN-ETSU company, Trefil E-505C^{®} and Trefil E-506C^{®} by the DOW-CORNING company, Gransil SR-CYC^{®}, SR DMF10^{®}, SR-DC556^{®}, SR 5CYC gel^{®}, SR DMF 10 gel^{®} and SR DC 556 gel^{®} by the GRANT INDUSTRIES company, SF 1204^{®} and JK 113^{®} by the GENERAL ELECTRIC company; ethylcellulose, such as the product sold under the name Ethocel^{®} by the DOW CHEMICAL company; polyamide-type polycondensates resulting from condensation between a dicarboxylic acid containing at least 32 carbon atoms and an alkylene diamine, and in particular ethylene diamine, in which the polymer comprises at least one terminal carboxylic acid group esterified or amidified with at least one monoalcohol or one monoamine containing from 12 to 30 carbon atoms, and linear and saturated, and in particular ethylenediamine/stearyl dilinoleate copolymers such as the product sold under the name Uniclear 100 VG^{®} by the ARIZONA CHEMICAL company; galactomannans containing from one to six, and in particular from two to four, hydroxyl groups per monosaccharide, substituted with a saturated or unsaturated alkyl chain, such as guar gum alkylated with C₁ to C₆ alkyl chains, and in particular C₁ to C₃ alkyl chains, and mixtures thereof. Block copolymers of the "diblock", "triblock" or "radial" type, of the polystyrene/polyisoprene or polystyrene/polybutadiene type, such as those sold under the name Luvitol HSB^{®} by the BASF company, of the polystyrene/copoly(ethylene-propylene) type, such as those sold under the name Kraton^{®} by the SHELL CHEMICAL Company or of the polystyrene/copoly(ethylenebutylene) type, blends of triblock and radial (star) copolymers in isododecane, such as those sold by the PENRECO company under the name Versagel^{®} for instance the mixture of butylene/ethylene/styrene triblock copolymer and of ethylene/propylene/styrene star copolymer in isododecane (Versagel M 5960).

Among the lipophilic gelling agents that can be used in a cosmetic composition of the invention, mention may also be made of esters of dextrin and of a fatty acid, such as dextrin palmitates, in particular such as those sold under the names Rheopearl TL^{®} or Rheopearl KL^{®} by the CHIBA FLOUR company, hydrogenated plant oils, such as hydrogenated castor oil, fatty alcohols, in particular C₈ to C₂₆, and more particularly C₁₂ to C₂₂, fatty alcohols, for instance myristyl alcohol, cetyl alcohol, stearyl alcohol and behenyl alcohol.

According to an embodiment, a composition may comprise thickeners in a content with respect to active material of from 0.01% to 40% by weight, especially from 0.1% to 20% by weight, in particular from 0.3% to 15% by weight, relative to the total weight of the composition.

According to a preferred embodiment, the composition includes at least one lipophilic thickener, in particular at least one modified hectorite, such as a hectorite modified by a C₁₀ to C₂₂ fatty acid ammonium chloride, advantageously in a content ranging from 0.1% to 5% by weight, in particular 0.5% to 2% by weight of active material with respect to the total weight of said composition

The composition may also include at least one powder material, in particular chosen from the powder dyes and fillers. According to a particular embodiment, the composition of the invention includes at least pigments and at least one filler. The powder content will generally range from 5% to 30% by weight, and in particular from 10% to 20% by weight with respect to the total weight of said composition.

### Dyes

A composition according to the invention may also include at least one dye, in particular a powder dye.

The dye(s) may be present with a content ranging from 2% to 25% by weight, in particular from 5% to 20% by weight, and preferably from 8% by 15% by weight with respect to the total of said composition.

A cosmetic composition in accordance with the invention may advantageously incorporate at least one dye selected from organic or inorganic dyes, in particular such as pigments or nacres conventionally used in cosmetic compositions, liposoluble or water-soluble coloring agents, materials with a specific optical effect, and mixtures thereof.

The term "pigments" should be understood to mean white or colored, inorganic or organic particles, which are insoluble in an aqueous solution and are intended for coloring and/or opacifying the resulting film.

The pigments may be present in a proportion of from 0.1% to 40% by weight, such as from 1% to 30% by weight, or from 5% to 15% by weight, relative to the total weight of the cosmetic composition.

As inorganic pigments that can be used in the invention, mention may be made of titanium, zirconium or cerium oxides, and also zinc, iron or chromium oxides, ferric blue, manganese violet, ultramarine blue and chromium hydrate. Preferably, the composition of the invention includes at least titanium oxides and iron oxides.

The pigment may also be a pigment having a structure that may be, for example, of sericite/brown iron oxide/titanium dioxide/silica type. Such a pigment is sold, for example, under the reference COVERLEAF NS or JS by the CHEMICALS AND CATALYSTS company and has a contrast ratio of around 30.

The dye may also comprise a pigment having a structure that may, for example, be of the type of silica microspheres containing iron oxide. An example of a pigment having this structure is sold by the MIYOSHI company under the name PC BALL PC-LL-100 P, and this pigment consists of silica microspheres containing yellow iron oxide.

The term "nacres" should be understood to mean iridescent or non-iridescent colored particles of any shape, which are in particular produced by certain mollusks in their shell or else are synthesized, and which exhibit a color effect by optical interference.

The nacres may be selected from pearlescent pigments such as titanium mica coated with iron oxide, titanium mica coated with bismuth oxychloride, titanium mica coated with chromium oxide, titanium mica coated with an organic dye, and pearlescent pigments based on bismuth oxychloride. This may also involve mica particles at the surface of which are superposed at least two successive layers of metal oxides and/or of organic dyes.

By way of example of nacres, mention may also be made of natural mica coated with titanium oxide, with iron oxide, with natural pigment or with bismuth oxychloride.

Among the nacres available on the market, mention may be made of the TIMICA, FLAMENCO and DUOCHROME nacres (based on mica) sold by the ENGELHARD company, the TIMIRON nacres sold MERCK company, the nacres based on mica, PRESTIGE, sold by the ECKART company and the nacres based on synthetic mica, SUNSHINE, sold by the SUN CHEMICAL company.

The nacres may more particularly possess a yellow, pink, red, bronze, orange, brown, gold and/or copper color or glint.

By way of illustration of nacres which can be used in the context of the invention, mention may, in particular, be made of the gold nacres sold, in particular, by the ENGELHARD company, under the name Brilliant gold 212G (Timica), Gold 222C (Cloisonne), Sparkle gold (Timica), Gold 4504 (Chromalite) and Monarch gold 233X (Cloisonne); the bronze nacres, sold, in particular, by the MERCK company under the name Bronze fine (17384) (Colorona) and Bronze (17353) (Colorona) and by the ENGELHARD company under the name Super bronze (Cloisonne); the orange nacres, in particular, sold by the ENGELHARD company under the name Orange 363C (Cloisonne) and Orange MCR 101 (Cosmica) and by the MERCK company under the name Passion orange (Colorona) and Matte orange (17449) (Microna); e brown-hued nacres sold in particular by the ENGELHARD company under the name Nu-antique copper 340XB (Cloisonne) and Brown CL4509 (Chromalite); the copper-glint nacres sold in particular by the ENGELHARD company under the name Copper 340A (Timica); the red-glint nacres sold in particular by the MERCK company under the name Sienna fine (17386) (Colorona); the yellow-glint nacres sold in particular by the ENGELHARD company under the name Yellow (4502) (Chromalite); the gold-glint red-hued nacres sold in particular by the ENGELHARD company under the name Sunstone G012 (Gemtone); the pink nacres sold in particular by the ENGELHARD company under the name Tan opal G005 (Gemtone); the gold-glint black nacres sold in particular by the ENGELHARD company under the name Nu-antique bronze 240 AB (Timica), the blue nacres sold in particular by the MERCK company under the name Matte blue (17433) (Microna), the silver-glint white nacres sold in particular by the MERCK company under the name Xirona Silver and the green-gold and pinkish orangish nacres sold in particular by the MERCK company under the name Indian summer (Xirona) and mixtures thereof.

The cosmetic composition according to the invention may also comprise water-soluble or liposoluble dyes. The liposoluble dyes are, for example, Sudan Red, DC Red 17, DC Green 6, β-carotene, soybean oil, Sudan brown, DC Yellow 11, DC Violet 2, DC Orange 5 and Quinoline Yellow. The water-soluble dyes are, for example, beetroot juice and caramel.

The cosmetic composition according to the invention may also contain at least one material with a specific optical effect.

This effect is different from a simple conventional hue effect, i.e. a unified and stabilized effect of the kind produced by conventional dyes, such as, for example, monochromatic pigments. For the purpose of the invention, the term "stabilized" signifies absence of an effect of variability of color with the angle of observation or in response to a temperature change.

For example, this material may be selected from particles having a metallic glint, goniochromatic coloring agents, diffracting pigments, thermochromatic agents, optical brighteners, and also fibers, in particular of the interference type. Of course, these various materials may be combined so as to provide the simultaneous manifestation of two effects, or even a new effect in accordance with the invention.

The metallic-glint particles that can be used in the invention are in particular selected from:
- particles of at least one metal and/or of at least one metal derivative,
- particles comprising a single-substance or multi-substance, organic or inorganic substrate, at least partially coated with at least one metal-glint layer comprising at least one metal and/or at least one metal derivative, and
- mixtures of said particles.

Among the metals that may be present in said particles, mention may, for example, be made of Ag, Au, Cu, Al, Ni, Sn, Mg, Cr, Mo, Ti, Zr, Pt, Va, Rb, W, Zn, Ge, Te, Se and mixtures or alloys thereof. Ag, Au, Cu, Al, Zn, Ni, Mo, Cr and mixtures or alloys thereof (for example, bronzes and brasses) are preferred metals.

The term "metal derivatives" denotes compounds derived from metals, in particular oxides, fluorides, chlorides and sulfides.

By way of illustration of these particles, mention may be made of aluminum particles, such as those sold under the names STARBRITE 1200 EAC^{®} by the SIBERLINE company and METALURE^{®} by the ECKART company.

Mention may also be made of metal powders of copper or of alloy mixtures, such as the references 2844 sold by the company RADIUM BRONZE, metal pigments, such as aluminum or bronze, for instance those sold under the name ROTOSAFE 700 of the ECKART company, the silica-coated aluminum particles sold under the name VISIONAIRE BRIGHT SILVER of the ECKART company and the metal alloy particles such as silica-coated bronze (copper and zinc alloy) sold under the name Visionaire Bright Natural Gold of the Eckart company.

The particles in question may also be particles comprising a glass substrate, such as those sold by the NIPPON SHEET GLASS company under the name MICROGLASS METASHINE.

The goniochromatic coloring agent may be selected, for example, from multilayer interference structures and liquid-crystal coloring agents.

Examples of symmetrical multilayer interference structures that may be used in compositions prepared in accordance with the invention are, for example, the following structures: Al/SiO₂/Al/SiO₂/Al, pigments having this structure being sold by the DUPONT DE NEMOURS company; Cr/MgF₂/Al/MgF₂/Cr, pigments having this structure being sold under the name CHROMAFLAIR by the FLEX company; MoS₂/SiO₂/Al/SiO₂/MoS₂; Fe₂O₃/SiO₂/Al/SiO₂/Fe₂O₃, and Fe₂O₃/SiO₂/Fe₂O₃/SiO₂/Fe₂O₃, pigments having this structure being sold under the name SICOPEARL by the BASF company; MoS₂/SiO₂/mica-oxyde/SiO₂/MoS₂; Fe₂O₃/SiO₂/mica-oxyde/SiO₂/Fe₂O₃; TiO₂/SiO₂/TiO₂ and TiO₂/Al₂O₃/TiO₂; SnO/TiO₂/SiO₂/TiO₂/SnO; Fe₂O₃/SiO₂/Fe₂O₃; SnO/mica/TiO₂/SiO₂/TiO₂/mica/SnO, pigments having this structure being sold under the name XIRONA by the MERCK company (Darmstadt). By way of example, these pigments may be the pigments with a silica/titanium oxide/tin oxide structure sold under the name XIRONA MAGIC by the MERCK company, pigments with a silica/brown iron oxide structure sold under the name XIRONA INDIAN SUMMER by the MERCK company and pigments with a silica/titanium oxide/mica/tin oxide structure sold under the name XIRONA CARRIBEAN BLUE by the MERCK company. Mention may also be made of the INFINITE COLORS pigments of the SHISEIDO company. Depending on the thickness and the nature of the various layers, various effects are obtained. Thus, with the structure Fe₂O₃/SiO₂/Al/ SiO₂/Fe₂O₃ the color changes from green-golden to red-gray for SiO₂ layers from 320 to 350nm; from red to golden for SiO₂ layers from 380 to 400nm; from violet to green for SiO₂ layers from 410 to 420nm; from copper to red for SiO₂ layers from 430 to 440nm.

By way of example of pigments with a polymeric multilayer structure, mention may be made of those sold by the 3M company under the name COLOR GLITTER.

Examples of liquid-crystal goniochromatic particles that may be used include those sold by the CHENIX company, and also the product sold under the name HELICONE^{®} HC by the Wacker company.

### Fillers

A composition according to the invention may also comprise at least one filler, of organic or inorganic nature, which makes it possible in particular to confer thereon additional properties of mattness, covering power and/or improved stability or staying power.

The filler content may range from 2% to 20% by weight, and in particular from 4% to 12% by weight, with respect to the total weight of said composition.

The term "filler" should be understood to mean colorless or white solid particles of any shape, which are in a form that is insoluble or dispersed in the medium of the composition. Inorganic or organic in nature, they make it possible to confer body or rigidity on the composition, and/or softness, and uniformity on the makeup.

The fillers used in the compositions according to the present invention may be of lamellar, globular or spherical form, or in the form of fibers or in any other intermediate form between these defined forms.

The fillers may or may not be surface-coated, and in particular they may be surface-treated with silicones, amino acids, fluorinated derivatives or any other substance that promotes the dispersion and compatibility of the filler in the composition.

As examples of inorganic fillers, mention may be made of talc, mica, silica, hollow silica microspheres, kaolin, calcium carbonate, magnesium carbonate, hydroxyapatite, boron nitride, glass or ceramic microcapsules, composites of silica and of titanium dioxide, such as the TSG series sold by Nippon Sheet Glass.

As examples of organic fillers, mention may be made of polyamide powder (Nylon^{®} Orgasol of Atochem), polyethylene powder, polymethyl methacrylate powder, polytetrafluoroethylene powder (Teflon), acrylic acid copolymer powder (Polytrap of the Dow Corning company), lauroyl lysine, hollow polymeric microspheres such as those of polyvinylidene/acrylonitrile chloride, for instance Expancel (Nobel Industry), hexamethylene diisocyanate/Trimethylol hexyllactone copolymer powder (Plastic Powder of Toshiki), silicone resin microbeads (Tospearl of Toshiba, for example) synthetic or natural micronized waxes, metal soaps derived from organic carboxylic acids having 8 to 22 carbon atoms, and preferably from 12 to 18 carbon atoms, for example, zinc stearate, magnesium stearate or lithium stearate, zinc laurate, magnesium myristate Polypore^{®} L 200 (Chemdal Corporation), cross-linked elastomeric organopolysiloxane powders coated with silicone resin, in particular silsesquioxane resin, as described for example in the patent US 5 538 793, polyurethane powders, in particular, cross-linked polyurethane powders including a copolymer, in which said copolymer includes hexyllactone trimethylol. In particular, it may be a polymer of hexamethylene diisocyanate/trimethylol hexyllactone. Such particles are in particular commercially available, for example under the name PLASTIC POWDER D-400^{®} or PLASTIC POWDER D-800^{®} of the TOSHIKI company and mixtures thereof.

According to a particular embodiment of the invention, the composition includes at least one cross-linked elastomeric organopolysiloxane powder coated with silicone resin. The presence of this filler also enables the composition of the invention to be thickened and/or gelled.

The cross-linked elastomeric organopolysiloxane powder(s) coated with silicone resin may be present in a content ranging from 2% to 12% by weight, advantageously from 4% to 10% by weight and preferably from 7% to 9% by weight with respect to the total weight of said composition.

In particular, mention may be made of cross-linked elastomeric organopolysiloxane powders coated with silicone resin, in particular silsesquioxane resin, as described for example in the patent US 5 538 793. Such elastomer powders are sold under the names KSP-100^{®}, KSP-101^{®}, KSP-102^{®}, KSP-103^{®}, KSP-104^{®} and KSP-105^{®} by the SHIN ETSU company; mention may also be made of cross-linked elastomeric organopolysiloxane powder coated with silicone resin such as hybrid silicone powders functionalized by fluoroalkyl groups, in particular sold under the name "KSP-200" by the Shin Etsu company; or hybrid silicone powders functionalized by phenyl groups, in particular sold under the name "KSP-300" by the Shin Etsu company.

### Additives

A cosmetic composition according to the invention may also further comprise any additive normally used in the field under consideration, for example selected from gums, anionic, cationic, amphoteric or nonionic surfactants, silicone surfactants, gums, resins, dispersants, semicrystalline polymers, antioxidants, essential oils, preservatives, fragrances, neutralizing agents, antiseptics, anti-UV protective agents, cosmetic active agents, such as vitamins, hydrating agents, emollients or collagen-protecting agents, and mixtures thereof.

A person skilled in the art can adjust the type and amount of additives present in the compositions according to the invention by means of routine operations, so that the cosmetic properties and the stability properties desired for these compositions are not affected by the additives.

A cosmetic composition of the invention may in particular be in the form of an anhydrous liquid product, an anhydrous gel, in the form of a stick or in the form of a soft paste. In particular, a cosmetic composition of the invention may be in the form of a liquid or fluid foundation, a hot-pour foundation, a body makeup product, a concealer or an eye shadow, or a makeup base.

A care composition according to the invention may, in particular, be a sun composition, a skincare cream, a serum or a deodorant.

Preferably, the composition according to the invention is in the form of a fluid foundation.

This invention also relates to the use, in a cosmetic composition for makeup and/or skincare, of at least one vinyl polymer having at least one carbosiloxane dendrimer-derived unit as defined above and a monoalcohol including from 2 to 8 carbon atoms in an amount of between 10% and 40% by weight with respect to the total weight of said composition, in order to increase the stability (in particular of the mattness) conferred by said composition and facilitate the application thereof.

### Mattness and mattness stability

The mattness and mattness stability may be measured by means of the protocol described below.

The mattness of a region of the skin, for example, of the face, is measured by means of a polarimetric camera, which is a black-and-white polarimetric imaging system, with which images with parallel-polarized (P) and cross-polarized (C) lighting are obtained.

By analyzing the image resulting from the subtraction of the two images (P-C), the shine is quantified by measuring the average gray level of the brightest 5% of the pixels corresponding to the shiny areas.

More specifically, the measurements are obtained on a panel of people, for example, a sample of 16 women, who are kept in an air-conditioned waiting room (22°C ± 2°C) for 15 minutes before the start of the test. They remove their makeup and an image of one of their cheeks is taken with the polarimetric camera. This image makes it possible to measure the shine at T0 before applying makeup. Approximately 100mg of cosmetic composition are then weighed in a watch glass, and applied, with bare fingers, to the half of the face on which the measurement at T0 was performed.

After a drying time of 15 minutes, an image of the made-up cheek is taken with the polarimetric camera. This image makes it possible to measure the shine just after applying makeup (Timm). The models then return to the air-conditioned room for 3 hours.

Finally, an image of the made-up cheek after 3 hours of waiting is taken with the polarimetric camera. This image makes it possible to measure the shine after 3 hours of wearing makeup (T3h).

The results are expressed by calculating the difference (Timm-T0), which measures the effect of the makeup. A negative value means that the makeup reduces the shine of the skin and that it is therefore mattifying.

The difference (T3h-Timm) measuring the stability of this effect is then calculated. The value obtained should be as low as possible, to signify that the mattness of the makeup does not change over time.

This invention also relates to a cosmetic treatment method including the application on the face of a composition as defined above.

This invention also relates to a method non-therapeutic makeup and/or skincare method including a step of applying, on the skin, at least one layer of a composition as defined above.

This invention also relates to a skin makeup method in which a composition as defined above is applied.

### EXAMPLES

### Influence de la nature of the polymer on mattness stability

The fluid foundation examples 1 and 2 show that the silicone dendrimer acrylate of the invention makes it possible to obtain better mattness stability properties than a silicone resin.

| | | Example 1 (Invention) | Example 2 (Comparative) |
|---|---|---|---|
| | | mass % | mass % |
| A1 | Isododecane | 6.00 | 6.00 |
| | Disteardimonium hectorite / propylene carbonate / isododecane (10/3/87) sold under the name BENTONE GEL ISD V by the Elementis company | 10.00 | 10.00 |
| | Phenyltrimethicone sold under the reference DC 556 by the Dow Corning company | 2.00 | 2.00 |
| | Ethyl hexyl methoxycinnamate | 3.00 | 3.00 |
| A2 | Butyl acrylate copolymer containing dendritic silicone side chains: Tri((Trimethylsiloxy)siloxyethyldimethylsiloxy)silylpropyl-methacrylate in the isododecane (40/60) sold under the reference Dow Corning FA 4002 ID by Dow Corning. | 5.00 | - |
| | Trimethyl siloxysilicate resin sold under the reference SR 1000 by the Momentive Performance Materials company. | - | 2.00 |
| | Isododecane | - | 3.00 |
| A3 | Cyclohexasiloxane | 4.00 | 4.00 |
| | Dimethicone copolyol sold under the reference KF 6017 by the Shin Etsu company | 1.50 | 1.50 |
| | Yellow iron oxide coated with aluminum stearoyl glutamate | 2.98 | 2.98 |
| | Red iron oxide coated with aluminum stearoyl glutamate | 0.94 | 0.94 |
| | Black iron oxide coated with aluminum stearoyl glutamate | 0.32 | 0.32 |
| | Titanium dioxide coated with aluminum stearoyl glutamate | 6.76 | 6.76 |
| A4 | Dodecamethylpentasiloxane | 20.75 | 20.75 |
| | Cyclohexasiloxane | 16.75 | 16.75 |
| | Vinyl dimethicone / Methicone Silsesquioxane cross-polymer sold under the reference KSP 100 by the Shin Etsu company | 8.00 | 8.00 |
| B | Denatured ethanol at 96° | 11.95 | 11.95 |
| C | Perfume | 0.05 | 0.05 |
| | TOTAL | 100% | 100% |

### Procedure example 1

The constituents of phases A1 and A2 are weighed into the main beaker and agitated, at ambient temperature, in the Rayneri mixer (200 to 300 rpm) for 15 minutes until homogenization.

Phase A3 is prepared separately by grinding three times, in a three-roll mill, the mixture of pigments, dimethicone copolyol and cyclohexasilixane.

This phase A3 is then added, while agitating, to the Rayneri mixer (400 to 500 rpm) for 10 minutes at ambient temperature.

Phase A4 is prepared separately by dispersing the KSP 100 in the mixture of the two oils, while agitating at ambient temperature, in the Rayneri mixer (500 rpm) for 10 minutes until homogenization.

This phase A4 is then added slowly, while agitating at ambient temperature, to the Rayneri (500 rpm) for 10 minutes.

The Rayneri agitation is then reduced to 200-300 rpm and phases B and C are added. After incorporation, the mixture is left to agitate for 5 more minutes at ambient temperature and then quickly packaged.

### Procedure example 2

The constituents of phase A1 are weighed into the main beaker and phase A2 is added.

The latter has been prepared beforehand by dispersing the silicone resin in isododecane while agitating with a Rayneri mixer equipped with a deflocculator (around 100 rpm).

The mixture is agitated at ambient temperature in the Rayneri mixer (200 to 300 rpm) for 15 minutes until homogenization.

Phase A3 is prepared separately by grinding three times, in a three-roll mill, the mixture of pigments, dimethicone copolyol and cyclohexasilixane.

This phase A3 is then added, while agitating, to the Rayneri mixer (400 to 500 rpm) for 10 minutes at ambient temperature.

Phase A4 is prepared separately by dispersing the KSP 100 in the mixture of the two oils, while agitating at ambient temperature, in the Rayneri mixer (500 rpm) for 10 minutes until homogenization.

This phase A4 is then added slowly, while agitating at ambient temperature, to the Rayneri (500 rpm) for 10 minutes.

The Rayneri agitation is then reduced to 200-300 rpm and phases B and C are added. After incorporation, the mixture is left to agitate for 5 more minutes at ambient temperature and then quickly packaged.

| | **Example 1 (Invention)** | **Example 2 (comparative)** |
|---|---|---|
| Nature of the polymer | Butyl acrylate copolymer containing dendritic silicone side chains: Tri((Trimethylsiloxy) siloxyethyldimethylsi loxy)silylpropylmethacrylate in the isododecane (40/60) sold under the reference Dow Corning FA 4002 ID by Dow Corning. | Trimethyl siloxysilicate resin sold under the reference SR 1000 by the Momentive Performance Materials company. |
| Mattness (Timm-T0) | -5.90 | -4.31 |
| Stability of the mattness (T3h-Timm) | 2.76 | 3.74 |

The mattness measurements are obtained according to the protocol described above.

These results show that the silicone dendrimer acrylate of the invention makes it possible to obtain the best mattness stability properties.

### Influence of the monoalcohol content

The foundation examples 3 and 4 show that an ethanol content greater than 10% enables better sensory properties to be obtained.

| | | Example 3 (Invention) | Example 4 (Comparative) |
|---|---|---|---|
| | | mass % | mass % |
| A1 | Isododecane | 3.00 | 3.00 |
| | Disteardimonium hectorite / propylene carbonate / isododecane (10/3/87) sold under the name BENTONE GEL ISD V by the Elementis company | 10.00 | 10.00 |
| | Phenyltrimethicone sold under the reference DC 556 by the Dow Corning company | 2.00 | 2.00 |
| | Ethyl hexyl methoxycinnamate | 3.00 | 3.00 |
| A2 | Butyl acrylate copolymer containing dendritic silicone side chains: Tri((Trimethylsiloxy)siloxyethyldimethylsiloxy)silylpropyl-methacrylate in the isododecane (40/60) sold under the reference Dow Corning FA 4002 ID by Dow Corning. | 8.00 | 8.00 |
| A3 | Cyclohexasiloxane | 4.00 | 4.00 |
| | Dimethicone copolyol sold under the reference KF 6017 by the Shin Etsu company | 1.50 | 1.50 |
| | Yellow iron oxide coated with aluminum stearoyl glutamate | 1.97 | 1.97 |
| | Red iron oxide coated with aluminum stearoyl glutamate | 0.59 | 0.59 |
| | Black iron oxide coated with aluminum stearoyl glutamate | 0.21 | 0.21 |
| | Titanium dioxide coated with aluminum stearoyl glutamate | 8.23 | 8.23 |
| A4 | Dodecamethylpentasiloxane | **20.75** | **23.73** |
| | Cyclohexasiloxane | **16.75** | **19.72** |
| | Vinyl dimethicone / Methicone Silsesquioxane cross-polymer sold under the reference KSP 100 by the Shin Etsu company | 8.00 | 8.00 |
| B | Denatured ethanol at 96° | **11.95** | **6.00** |
| C | Perfume | 0.05 | 0.05 |
| | TOTAL | 100% | 100% |

### Procedure

The constituents of phases A1 and A2 are weighed into the main beaker and agitated, at ambient temperature, in the Moritz mixer (3500 rpm) for 15 minutes until homogenization.

Phase A3 is prepared separately by grinding three times, in a three-roll mill, the mixture of pigments, dimethicone copolyol and cyclohexasilixane.

This phase A3 is then added, while agitating, to the Moritz mixer (3500 rpm) for 10 minutes at ambient temperature.

Phase A4 is prepared separately by dispersing the KSP 100 in the mixture of the two oils, while agitating at ambient temperature, in the Rayneri mixer (500 rpm) for 10 minutes until homogenization.

This phase A4 is then added slowly, while agitating at ambient temperature, to the Moritz mixer and while increasing the agitation speed from 3500 rpm to 4000 rpm. It is then left to agitate for 10 minutes at 3500 rpm.

The Moritz agitation is then reduced to 1500 rpm and phases B and C are added while progressively increasing the agitation speed from 1500 to 2500 rpm. After incorporation, the mixture is left to agitate for 5 more minutes at ambient temperature at 2500 rpm and then quickly packaged.

### Sensory evaluation

We asked a panel of 5 women at the Laboratory, between the ages of 20 and 45 years, to apply one of the two foundations on each half of their face.

This evaluation showed found that the foundation of example 3 (invention) is less oily and less greasy on application. It leads to a more uniform, softer, less tacky and less oily makeup result than that obtained with the foundation of example 4 (comparative).

In the end, the women on the panel prefer the foundation of example 3 (invention).

The following foundation examples 5 and 6 show that an ethanol content lower than 40% enables better sensory properties to be obtained.

| | | Example 5 (Invention) | Example 6 (Comparative) |
|---|---|---|---|
| | | % mass | % mass |
| A1 | Isododecane | 3.00 | 3.00 |
| | Phenyltrimethicone sold under the reference DC 556 by the Dow Corning company | 2.00 | 2.00 |
| | Ethyl hexyl methoxycinnamate | 3.00 | 3.00 |
| | Butyl acrylate copolymer containing dendritic silicone side chains: Tri((Trimethylsiloxy)siloxyethyldimethylsiloxy)silylpropyl-methacrylate in the isododecane (40/60) sold under the reference Dow Corning FA 4002 ID by Dow Corning. | 8.00 | 8.00 |
| | Dodecamethylpentasiloxane | **9.23** | **4.23** |
| | Cyclohexasiloxane | 5.00 | - |
| A2 | Vinyl dimethicone / Methicone Silsesquioxane cross-polymer sold under the reference KSP 100 by the Shin Etsu company | 8.00 | 8.00 |
| A3 | Cyclohexasiloxane | 4.22 | 4.22 |
| | Dimethicone copolyol sold under the reference KF 6017 by the Shin Etsu company | 1.50 | 1.50 |
| | Yellow iron oxide coated with aluminum stearoyl glutamate | 1.97 | 1.97 |
| | Red iron oxide coated with aluminum stearoyl glutamate | 0.59 | 0.59 |
| | Black iron oxide coated with aluminum stearoyl glutamate | 0.21 | 0.21 |
| | Titanium dioxide coated with aluminum stearoyl glutamate | 8.23 | 8.23 |
| A4 | Disteardimonium hectorite / propylene carbonate / isododecane (10/3/87) sold under the name BENTONE GEL ISD V by the Elementis company | 10.00 | 10.00 |
| B | Denatured ethanol at 96° | **35.00** | **45.00** |
| C | Perfume | 0.05 | 0.05 |
| | TOTAL | 100% | 100% |

### Procedure

The constituents of phase A1 are weighed into the main beaker and phase A2 is sprinkled while agitating, at ambient temperature, in the Rayneri mixer equipped with a deflocculator (1000 rpm) for 15 minutes until homogenization.

Phase A3 is prepared separately by grinding three times, in a three-roll mill, the mixture of pigments, dimethicone copolyol and cyclohexasilixane.

This phase A3 is then added into the main beaker as well as phases A4, B and C, while agitating, to the Moritz mixer (2500 rpm) at ambient temperature until homogenization.

### Sensory evaluation

We asked a panel of 4 women at the Laboratory, between the ages of 20 and 45 years, to apply one of the two foundations on each half of their face.

This evaluation showed found that the foundation of example 5 (invention) has a more creamy and more glidant texture.

This foundation is easier to be applied onto the skin and its play-time is longer. Furthermore, this foundation dries less quickly.

No discomfort has been noticed for the foundation of example 5 whereas for the foundation of example 6 the women felt a slight tingling.

In the end, the women on the panel prefer the foundation of example 5 (invention).

## Claims

1. Composition including a physiologically acceptable medium containing at least one vinyl polymer having at least one carbosiloxane dendrimer-derived unit and one or more monoalcohol(s) containing 2 to 8 carbon atoms, in which the amount of monoalcohol(s) ranges from 10% to 40%, in particular 10% to 20%, more particularly 10% to 15%, and preferably 11% to 15%, by weight with respect to the total weight of said composition.

2. Composition according to claim 1, in which the vinyl polymer having at least one carbosiloxane dendrimer-derived unit has a molecular side chain containing a carbosiloxane dendrimer structure and results from the polymerization of:
(A) from 0 to 99.9 parts per weight of a vinyl polymer; and
(B) from 100 to 0.1 parts per weight of a carbosiloxane dendrimer of the following formula (I): 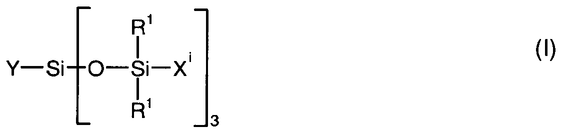 in which:
- R¹ represents an aryl group having from 5 to 10 carbon atoms or an alkyl group having from 1 to 10 carbon atoms;
- X¹ represents a silylalkyl group which, when i = 1, is represented by the formula (II): 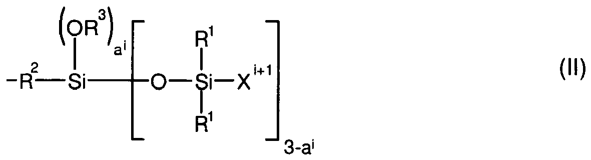 in which:
. R¹ is as defined above in formula (I),
. R² represents an alkylene group having from 2 to 10 carbon atoms,
. R³ represents an alkyl group having from 1 to 10 carbon atoms,
. Xⁱ⁺ⁱ is chosen from: a hydrogen atom, an alkyl group having from 1 to 10 carbon atoms, an aryl group having from 5 to 10 carbon atoms and a silylalkyl group as defined above of formula (II) with i = i + 1,
. i is an integer from 1 to 10 that represents the generation of said silylalkyl group, and
. aⁱ is an integer from 0 to 3;
- Y represents a radical-polymerizable organic group selected from:
. organic groups containing a methacrylic group or an acrylic group and which are represented by the formulas: 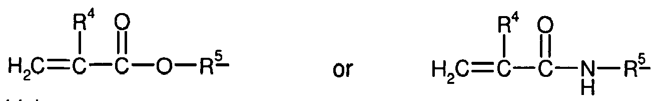 in which:
* R⁴ represents a hydrogen atom or an alkyl group having from 1 to 10 carbon atoms; and
* R⁵ represents an alkylene group having from 1 to 10 carbon atoms, and
. organic groups containing a styryl group and which are represented by the formula:
in which: 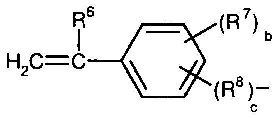
* R⁶ represents a hydrogen atom or an alkyl group having from 1 to 10 carbon atoms;
* R⁷ represents an alkyl group having from 1 to 10 carbon atoms;
* R⁸ represents an alkylene group having from 1 to 10 carbon atoms;
* b is an integer from 0 to 4; and
* c is 0 or 1, so that if c is 0, -(R⁸)_{c}- represents a bond.

3. Composition according to claim 1 or 2, in which the carbosiloxane dendrimer is represented by the following formula: 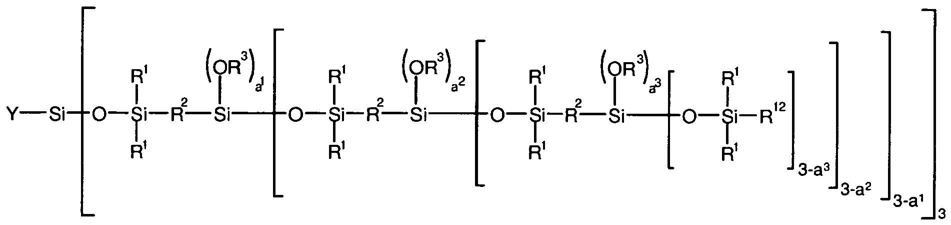 in which:
. Y, R¹, R² and R³ are as defined in claim 2;
. a¹, a² and a³ respond to the definition of aⁱ according to claim 2; and
. R¹² is H, an aryl group having from 5 to 10 carbon atoms or an alkyl group having from 1 to 10 carbon atoms.

4. Composition according to any one of claims 1 to 3, in which the carbosiloxane dendrimer is represented by one of the following formulas:

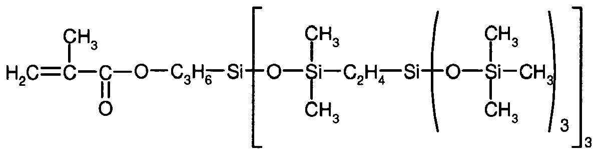

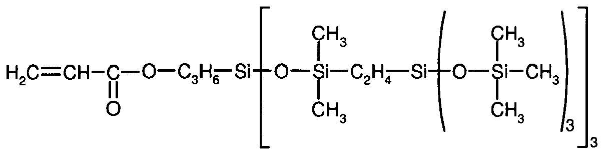

5. Composition according to any one of claims 1 to 4, in which the vinyl polymer having at least one carbosiloxane dendrimer-derived unit is present in a content of active material ranging from 0.5% to 20% by weight, in particular from 1% to 15%, more specifically from 2% to 10% and preferably from 3% to 5% by weight with respect to the total weight of said composition.

6. Composition according to any one of claims 1 to 5, including an alcoholic or hydro-alcoholic phase and a fatty phase, in which said alcoholic or hydro-alcoholic phase contains the monoalcohol and said fatty phase contains the vinyl polymer having at least one carbosiloxane dendrimer-derived unit.

7. Composition according to any one of claims 1 to 6, in which the fatty phase includes at least one volatile oil and/or at least one non-volatile oil.

8. Composition according to any one of claims 1 to 7, in which the fatty phase includes from 40% to 100%, preferably from 60% to 98%, and more particularly from 80% to 95% by weight of volatile oil(s) with respect to the total weight of the fatty phase, and less than 60%, preferably from 1% to 40%, and more particularly from 2% to 20% by weight of non-volatile oil(s) with respect to the total weight of the fatty phase.

9. Composition according to any one of claims 1 to 8, in which the fatty phase represents from 25% to 85%, preferably from 40% to 75%, and more particularly from 50% to 70%, by weight with respect to the total weight of said composition.

10. Composition according to any one of claims 1 to 9, in which the monoalcohol is ethanol.

11. Composition according to any one of claims 1 to 10, also including a powder material, in particular chosen from powder dyes and fillers.

12. Composition according to any one of claims 1 to 11, including a thickener or gelling agent and/or a lipophilic structuring agent.

13. Composition according to any one of claims 1 to 12, in the form of an anhydrous composition or including less than 5% by weight of water, more preferably less than 2% by weight of water, even more preferably less than 1% by weight of water, with respect to the total weight of said composition, in which said composition is in particular in the form of a fluid foundation.

14. Use, in a cosmetic composition for makeup and/or skin care, of at least one vinyl polymer having at least one carbosiloxane dendrimer derivative and at least one monoalcohol including 2 to 8 carbon atoms in an amount of between 10% and 40% by weight with respect to the total weight of said composition, in order to increase the stability of the mattness conferred by said composition.

15. Method for cosmetic treatment including the application, on the skin, of a composition according to any one of claims 1 to 13.

## Patentansprüche

1. Zusammensetzung, die ein physiologisch akzeptables Medium beinhaltet, das mindestens ein Vinylpolymer mit mindestens einer von Carbosiloxan-Dendrimer abgeleiteten Einheit und einen oder mehrere Monoalkohole, der bzw. die 2 bis 8 Kohlenstoffatome enthält bzw. enthalten, enthält, wobei die Menge an Monoalkohol(en) von 10 Gew.-% bis 40 Gew.-%, insbesondere 10 Gew.-% bis 20 Gew.-%, mehr bevorzugt 10 Gew.-% bis 15 Gew.-% und vorzugsweise 11 Gew.-% bis 15 Gew.-% in Bezug auf das Gesamtgewicht der besagten Zusammensetzung reicht.

2. Zusammensetzung nach Anspruch 1, wobei das Vinylpolymer mit mindestens einer von Carbosiloxan-Dendrimer abgeleiteten Einheit eine Molekülseitenkette aufweist, die eine Carbosiloxan-Dendrimer-Struktur enthält, und aus der Polymerisation der folgenden resultiert:
(A) von 0 bis 99,9 Gewichtsteile eines Vinylpolymers; und
(B) von 100 bis 0,1 Gewichtsteile eines Carbosiloxan-Dendrimers der folgenden Formel (I): 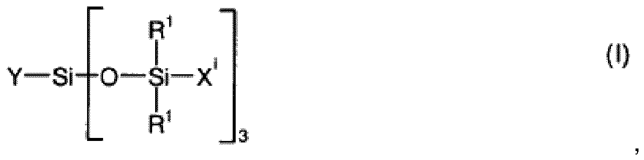 wobei:
- R¹ für eine Arylgruppe mit 5 bis 10 Kohlenstoffatomen oder eine Alkylgruppe mit 1 bis 10 Kohlenstoffatomen steht;
- Xⁱ für eine Silylalkylgruppe steht, die, wenn i = 1, durch die Formel (II) dargestellt wird: 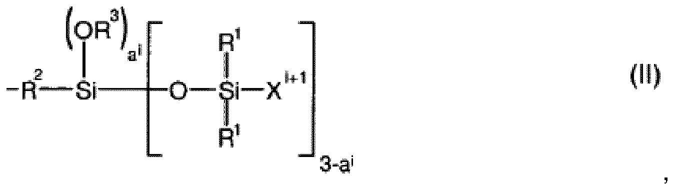 wobei:
. R¹ wie oben in Formel (I) definiert ist,
. R² für eine Alkylengruppe mit 2 bis 10 Kohlenstoffatomen steht,
. R³ für eine Alkylgruppe mit 1 bis 10 Kohlenstoffatomen steht,
. Xⁱ⁺¹ aus folgenden ausgewählt ist: einem Wasserstoffatom, einer Alkylgruppe mit 1 bis 10 Kohlenstoffatomen, einer Arylgruppe mit 5 bis 10 Kohlenstoffatomen und einer wie oben definierten Silylalkylgruppe von Formel (II), wobei i = i + 1,
. i eine ganze Zahl von 1 bis 10 ist, die für die Erzeugung der besagten Silylalkylgruppe steht, und
. aⁱ eine ganze Zahl von 0 bis 3 ist;
- Y für eine radikalpolymerisierbare organische Gruppe steht, die aus folgenden ausgewählt ist:
. organischen Gruppen, die eine Methacrylgruppe oder eine Acrylgruppe enthalten und die durch die folgenden Formeln dargestellt werden: 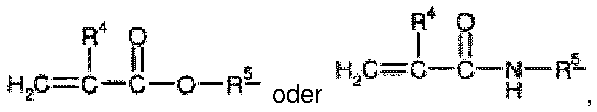 wobei:
* R⁴ für ein Wasserstoffatom oder eine Alkylgruppe mit 1 bis 10 Kohlenstoffatomen steht; und
* R⁵ für eine Alkylengruppe mit 1 bis 10 Kohlenstoffatomen steht, und
. organischen Gruppen, die eine Styrylgruppe enthalten und die durch die folgende Formel dargestellt werden: 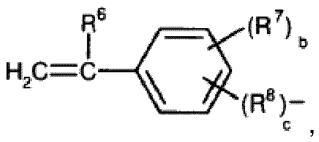 wobei:
* R⁶ für ein Wasserstoffatom oder eine Alkylgruppe mit 1 bis 10 Kohlenstoffatomen steht;
* R⁷ für eine Alkylgruppe mit 1 bis 10 Kohlenstoffatomen steht;
* R⁸ für eine Alkylengruppe mit 1 bis 10 Kohlenstoffatomen steht;
* b eine ganze Zahl von 0 bis 4 ist; und
* c 0 oder 1 ist, so dass, wenn c 0 ist, -(R⁸)_{c}- für eine Bindung steht.

3. Zusammensetzung nach Anspruch 1 oder 2, wobei das Carbosiloxan-Dendrimer durch die folgende Formel dargestellt wird: 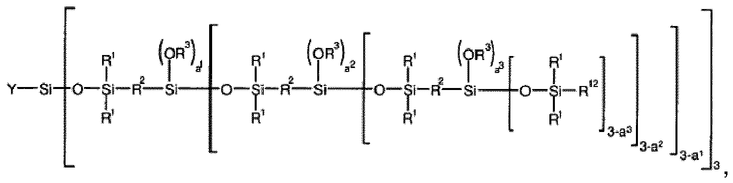 wobei:
. Y, R¹, R² und R³ wie in Anspruch 2 definiert sind;
. a¹, a² und a³ der Definition von aⁱ nach Anspruch 2 entsprechen; und
. R ¹² H, eine Arylgruppe mit 5 bis 10 Kohlenstoffatomen oder eine Alkylgruppe mit 1 bis 10 Kohlenstoffatomen ist.

4. Zusammensetzung nach einem der Ansprüche 1 bis 3, wobei das Carbosiloxan-Dendrimer durch eine der folgenden Formeln dargestellt wird:

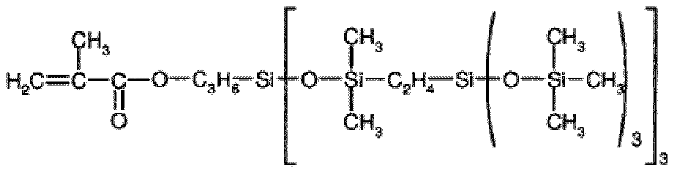

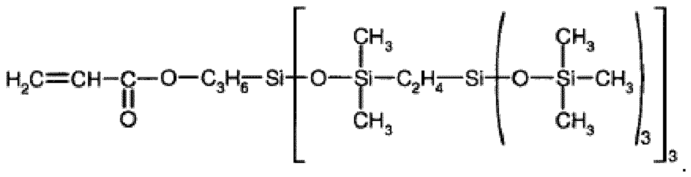

5. Zusammensetzung nach einem der Ansprüche 1 bis 4, wobei das Vinylpolymer mit mindestens einer von Carbosiloxan-Dendrimer abgeleiteten Einheit in einem Gehalt von aktivem Material vorliegt, der von 0,5 Gew.-% bis 20 Gew.-%, insbesondere von 1 Gew.-% bis 15 Gew.-%, spezifischer von 2 Gew.-% bis 10 Gew.-% und vorzugsweise von 3 Gew.-% bis 5 Gew.-% in Bezug auf das Gesamtgewicht der besagten Zusammensetzung reicht.

6. Zusammensetzung nach einem der Ansprüche 1 bis 5, die eine alkoholische oder hydroalkoholische Phase und eine Fettphase beinhaltet, wobei die besagte alkoholische oder hydroalkoholische Phase den Monoalkohol enthält und die besagte Fettphase das Vinylpolymer mit mindestens einer von Carbosiloxan-Dendrimer abgeleiteten Einheit enthält.

7. Zusammensetzung nach einem der Ansprüche 1 bis 6, wobei die Fettphase mindestens ein flüchtiges Öl und/oder mindestens ein nichtflüchtiges Öl beinhaltet.

8. Zusammensetzung nach einem der Ansprüche 1 bis 7, wobei die Fettphase von 40 Gew.-% bis 100 Gew.-%, vorzugsweise von 60 Gew.-% bis 98 Gew.-% und ganz besonders von 80 Gew.-% bis 95 Gew.-% eines oder mehrerer flüchtiger Öle in Bezug auf das Gesamtgewicht der Fettphase und weniger als 60 Gew.-%, vorzugsweise von 1 Gew.-% bis 40 Gew.-% und ganz besonders von 2 Gew.-% bis 20 Gew.-% eines oder mehrerer nichtflüchtiger Öle in Bezug auf das Gesamtgewicht der Fettphase beinhaltet.

9. Zusammensetzung nach einem der Ansprüche 1 bis 8, wobei die Fettphase von 25 Gew.-% bis 85 Gew.-%, vorzugsweise von 40 Gew.-% bis 75 Gew.-% und ganz besonders von 50 Gew.-% bis 70 Gew.-% in Bezug auf das Gesamtgewicht der besagten Zusammensetzung darstellt.

10. Zusammensetzung nach einem der Ansprüche 1 bis 9, wobei der Monoalkohol Ethanol ist.

11. Zusammensetzung nach einem der Ansprüche 1 bis 10, die außerdem ein Pulvermaterial beinhaltet, das insbesondere aus Pulverfarbstoffen und -füllstoffen ausgewählt ist.

12. Zusammensetzung nach einem der Ansprüche 1 bis 11, die einen Verdicker oder ein Geliermittel und/oder ein lipophiles Strukturiermittel beinhaltet.

13. Zusammensetzung nach einem der Ansprüche 1 bis 12 in der Form einer wasserfreien Zusammensetzung oder weniger als 5 Gew.-% Wasser, mehr bevorzugt weniger als 2 Gew.-% Wasser, noch mehr bevorzugt weniger als 1 Gew.-% Wasser in Bezug auf das Gesamtgewicht der besagten Zusammensetzung beinhaltend, wobei die Zusammensetzung insbesondere in der Form einer flüssigen Foundation ist.

14. Verwendung, in einer kosmetischen Zusammensetzung für Makeup und/oder Hautpflege, mindestens eines Vinylpolymers mit mindestens einem Carbosiloxan-Dendrimer-Derivat und mindestens eines Monoalkohols, der 2 bis 8 Kohlenstoffatome beinhaltet, in einer Menge zwischen 10 Gew.-% und 40 Gew.-% in Bezug auf das Gesamtgewicht der besagten Zusammensetzung, um die Stabilität der Mattheit, die von der besagten Zusammensetzung verliehen wird, zu erhöhen.

15. Verfahren zur kosmetischen Behandlung, das das Auftragen einer Zusammensetzung nach einem der Ansprüche 1 bis 13 auf die Haut beinhaltet.

## Revendications

1. Composition comprenant un milieu physiologiquement acceptable contenant au moins un polymère vinylique ayant au moins un motif dérivé de dendrimère carbosiloxane et un ou plusieurs mono-alcool(s) comprenant de 2 à 8 atomes de carbone, dans laquelle la quantité en mono-alcool(s) est comprise de 10% à 40%, de particulier de 10% à 20%, plus particulièrement de 10% à 15%, et de préférence de 11 % à 15%, en poids par rapport au poids total de ladite composition.

2. Composition selon la revendication 1, dans laquelle le polymère vinylique ayant au moins un motif dérivé de dendrimère carbosiloxane possède une chaîne moléculaire latérale contenant une structure de dendrimère carbosiloxane et est issu de la polymérisation de :
(A) de 0 à 99,9 parties en poids d'un monomère vinylique ; et
(B) de 100 à 0,1 parties en poids d'un dendrimère carbosiloxane de formule suivante (I) : 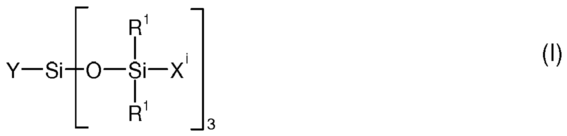 dans laquelle :
- R¹ représente un groupe aryle de 5 à 10 atomes de carbone ou un groupe alkyle de 1 à 10 atomes de carbone ;
- X' représente un groupe silylalkyle qui, lorsque i = 1, est représenté par la formule (II) : 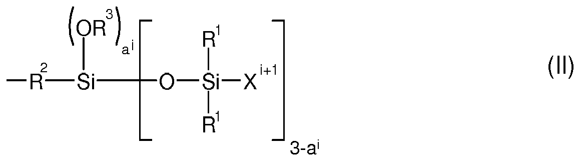 dans laquelle :
. R¹ est tel que défini ci-dessus dans la formule (I),
. R² représente un radical alkylène de 2 à 10 atomes de carbone,
. R³ représente un groupe alkyle de 1 à 10 atomes de carbone,
. Xⁱ⁺ⁱ est choisi parmi : un atome d'hydrogène, un groupe alkyle de 1 à 10 atomes de carbone, un groupe aryle de 5 à 10 atomes de carbone et un groupe silylalkyle défini ci-dessus de formule (II) avec i = i + 1,
. i est un nombre entier de 1 à 10 qui représente la génération dudit groupe silylalkyle, et
. aⁱ est un nombre entier de 0 à 3 ;
- Y représente un groupe organique polymérisable à l'aide de radicaux choisis parmi :
. des groupes organiques contenant un groupe méthacrylique ou un groupe acrylique, lesdits groupes organiques étant représentés par les formules : 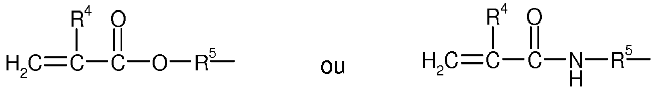 dans lesquelles :
* R⁴ représente un atome d'hydrogène ou un groupe alkyle de 1 à 10 atomes de carbone ; et
* R⁵ représente un groupe alkylène de 1 à 10 atomes de carbone ; et
. des groupes organiques contenant un groupe styryle de formule : 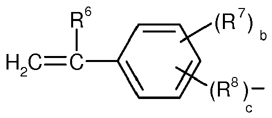 dans laquelle :
* R⁶ représente un atome d'hydrogène ou un groupe alkyle de 1 à 10 atomes de carbone ;
* R⁷ représente un groupe alkyle de 1 à 10 atomes de carbone ;
* R⁸ représente un groupe alkylène de 1 à 10 atomes de carbone ;
* b est un nombre entier de 0 à 4 ; et
* c vaut 0 ou 1, de sorte que si c vaut 0, -(R⁸)_{c}- représente une liaison.

3. Composition selon la revendication 1 ou 2, dans laquelle le dendrimère carbosiloxane est représenté par la formule suivante : 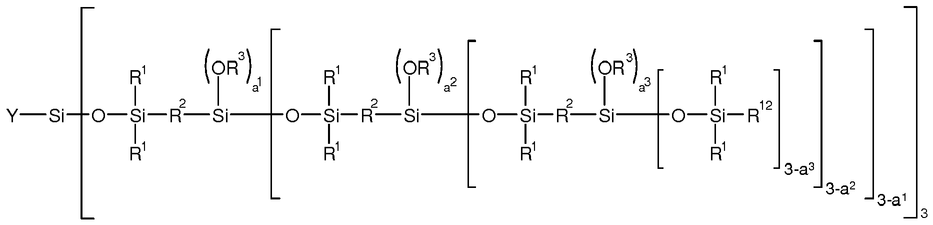 dans laquelle :
. Y, R¹, R² et R³ sont tels que définis dans la revendication 2 ;
. a¹, a² et a³ répondent à la définition de aⁱ selon la revendication 2 ; et
. R¹² est H, un groupe aryle de 5 à 10 atomes de carbone ou un groupe alkyle de 1 à 10 atomes de carbone.

4. Composition selon l'une quelconque des revendications 1 à 3, dans laquelle le dendrimère carbosiloxane est représenté par l'une des formules suivantes :

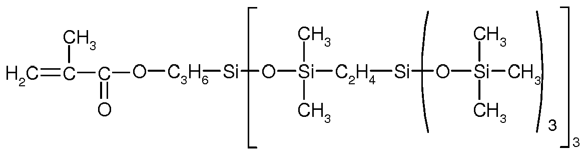

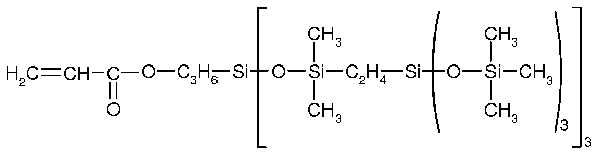

5. Composition selon l'une quelconque des revendications 1 à 4, dans laquelle le polymère vinylique ayant au moins un motif dérivé de dendrimère carbosiloxane est présent en une teneur en matière active comprise de 0,5% à 20%, de particulier de 1% à 15%, plus particulièrement de 2% à 10%, et de préférence de 3% à 5%, en poids par rapport au poids total de ladite composition.

6. Composition selon l'une quelconque des revendications 1 à 5, comprenant une phase alcoolique ou hydro-alcoolique et une phase grasse, ladite phase alcoolique ou hydro-alcoolique contenant le mono-alcool et ladite phase grasse contenant le polymère vinylique ayant au moins un motif dérivé de dendrimère carbosiloxane.

7. Composition selon l'une quelconque des revendications 1 à 6, dans laquelle la phase grasse comprend au moins une huile volatile et/ou au moins une huile non volatile.

8. Composition selon l'une quelconque des revendications 1 à 7, dans laquelle la phase grasse comprend de 40% à 100%, de préférence de 60% à 98%, et plus particulièrement de 80% à 95% en poids d'huile(s) volatile(s) par rapport au poids total de la phase grasse, et moins de 60%, de préférence de 1% à 40%, et plus particulièrement de 2% à 20% en poids d'huile(s) non volatile(s) par rapport au poids total de la phase grasse.

9. Composition selon l'une quelconque des revendications 1 à 8, dans laquelle la phase grasse représente de 25% à 85%, de préférence de 40% à 75%, et plus particulièrement de 50% à 70%, en poids par rapport au poids total de ladite composition.

10. Composition selon l'une quelconque des revendications 1 à 9, dans laquelle le mono-alcool est l'éthanol.

11. Composition selon l'une quelconque des revendications 1 à 10, comprenant en outre une matière pulvérulente, en particulier choisie parmi les matières colorantes pulvérulentes et les charges.

12. Composition selon l'une quelconque des revendications 1 à 11, comprenant un agent épaississant ou gélifiant et/ou un agent structurant lipophile.

13. Composition selon l'une quelconque des revendications 1 à 12, sous la forme d'une composition anhydre ou comprenant moins de 5% en poids d'eau, plus préférentiellement moins de 2% en poids d'eau, encore plus préférentiellement moins de 1% en poids d'eau, par rapport au poids total de ladite composition, ladite composition étant notamment sous la forme d'un fond de teint fluide.

14. Utilisation, dans une composition cosmétique pour le maquillage et/ou le soin de la peau, d'au moins un polymère vinylique ayant au moins un motif dérivé de dendrimère carbosiloxane et d'au moins un mono-alcool comprenant de 2 à 8 atomes de carbone en une quantité comprise de 10% à 40% en poids par rapport au poids total de ladite composition, pour augmenter la tenue de la matité conférée par ladite composition.

15. Procédé de traitement cosmétique comprenant l'application sur la peau d'une composition selon l'une quelconque des revendications 1 à 13.
